# EUROPEAN PATENT APPLICATION

(11) **EP 4 112 647 A1**
(43) Date of publication of application: **04.01.2023**
(21) Application number: 21760510.4
(22) Date of filing: 26.02.2021
(51) Int. Cl.: C07K 16/46, C12N 15/13, C12N 15/63, A61K 38/16, A61P 35/00

(54) **ANTI-CD47/ANTI-PD-L1 ANTIBODY AND APPLICATIONS THEREOF**

(30) Priority: 28.02.2020 CN 202010128900
(71) Applicant: Nanjing Sanhome Pharmaceutical Co., Ltd., Nanjing Jiangsu 210038 (CN)
(72) Inventor: XIAO, Yang, Nanjing, Jiangsu 210038 (CN); ZHU, Yongqiang, Nanjing, Jiangsu 210038 (CN); ZHAO, Liwen, Nanjing, Jiangsu 210038 (CN); WU, Wenming, Nanjing, Jiangsu 210038 (CN); ZHOU, Guo, Nanjing, Jiangsu 210038 (CN); LUO, Cheng, Nanjing, Jiangsu 210038 (CN); LI, Xue, Nanjing, Jiangsu 210038 (CN)
(74) Representative: ABG Intellectual Property Law, S.L.
(86) International application number: PCT/CN2021/078132
(87) International publication number: WO 2021/170082

(57) **Abstract**

Provided are an anti-CD47/anti-PD-L1 antibody, a pharmaceutical composition of the anti-CD47/anti-PD-L1 antibody, and applications thereof. The anti-CD47/anti-PD-L1 antibody provides antitumor activity, is free of significant red blood cell toxicity, and is applicable in preparing an antitumor medicament.

## Description

### CROSS REFERENCE TO RELATED APPLICATIONS

This is a European National Phase application based upon PCT Application No. PCT/CN2021/078132 filed on February 26, 2021, which claims the priority of Chinese Patent Application No. 202010128900.X filed on February 28, 2020 and titled with "ANTI-CD47/ANTI-PD-L1 ANTIBODY AND APPLICATIONS THEREOF".

### FIELD

The present disclosure relates to the technical field of antibody drugs, and in particular, to an anti-CD47/anti-PD-L1 antibody, a pharmaceutical composition comprising the anti-CD47/anti-PD-L1 antibodies, and uses thereof.

### BACKGROUND

CD47 protein, also known as integrin-associated protein (IAP), is a five-span transmembrane glycoprotein belonging to the IgG superfamily and widely expressed in different tissues and cells. CD47 can bind to the ligand TSP-1 or SIRPα to regulate different cellular functions, including migration, adhesion and apoptosis of cells, axon extension, cytokine production as well as T cell activation. SIRPα is a transmembrane protein containing a typical immunoreceptor tyrosine-based inhibitory motif (ITIM), and mainly expressed on the surface of the cell membrane of myeloid hematopoietic cells, such as macrophages, dendritic cells, etc. The binding of CD47 to SIRPα leads to phosphorylation of ITIMs and consequent recruitment of SHP-1/SHP-2, which further inhibit the accumulation of myosin IIA in phagocytic synapses, and ultimately inhibit the phagocytic function of phagocytes.

The "immune escape" of tumor cells is considered as the main mechanism of tumorigenesis, tumor development and drug resistance. Through high expression of CD47 molecules, which interact with SIRPα on the surface of macrophages, tumor cells can significantly inhibit the phagocytic activity of macrophages and avoid being swallowed by macrophages. When the binding of CD47 to SIRPα is blocked, the immune suppression or immune tolerance caused by tumor can be eliminated, and tumor cells can be effectively killed. This provides a very powerful theoretical basis for targeting CD47 as a target of targeted tumor immunotherapy.

In recent years, numerous studies have been conducted on various treatments targeting CD47/SIRPα signaling pathway at home and abroad. Among them, antibodies blocking CD47 are believed to be the most promising tumor treatment regimen. The effectiveness of monoclonal antibodies blocking human CD47 have been confirmed in diverse preclinical models. However, since red blood cells and platelets also express CD47 molecules, when the antibodies block the interaction between CD47 and SIRPα, it is likely that these cells may lose the protection of "don't eat me" signal and thereby be subjected to phagocytosis by macrophages. Therefore, the side effects of anti-CD47 antibody, such as degradation of platelets, agglutination of red blood cells, depletion of red blood cells, and anemia, etc. should be considered and avoided when applying anti-CD47 antibody.

Programmed death ligand 1 (PD-L1), also known as cluster of differentiation 274 (CD274) or B7 homolog 1 (B7-H1), is a 40 kDa type I transmembrane protein that plays a major role in suppressing the immune system during specific events such as pregnancy, tissue allotransplantation, autoimmune diseases and other disease states such as hepatitis. Binding of PD-L1 to PD-1 or B7.1 transmits an inhibitory signal that reduces the proliferation of CD8⁺ T cells in lymph nodes, and supplementation of PD-1 is also able to control the accumulation of T cells specific for foreign antigens in lymph nodes through apoptosis mediated by the gene Bcl-2.

The up-regulation of PD-L1 has been shown to allow cancers to evade the host immune system. Analysis of tumor samples from patients with renal cell carcinoma reveals that high PD-L1 expression in tumors was associated with increased tumor aggressiveness and increased risk of death. Many PD-L1 inhibitors are in development as immuno-oncology therapies and are showing promising results in clinical trials.

The CD47-SIRPα signaling pathway not only activates innate immunity, but further kills tumors in the way that macrophages present tumor antigens to CD8+ T and CD4+ T cells to promote T cell activation. Therefore, it is possible to develop a bifunctional fusion protein targeting CD47 and PD-L1 comprising a CD47-binding portion and a PD-L1-binding portion, which can block not only the binding of PD-L1 to PD-1 but also the binding of CD47 to SIRPα, and thus bridge the innate and adaptive immune signaling pathways, resulting in better antitumor activity, tumor targeting and lower toxicity on red blood cells.

### SUMMARY

The present disclosure provides an anti-CD47/anti-PD-L1 antibody comprising an anti-CD47 antibody or antigen-binding fragment thereof and an anti-PD-L1 antibody or antigen-binding fragment thereof, wherein the anti-CD47 antibody or antigen-binding fragment thereof comprises a first heavy chain variable region and/or a first light chain variable region, wherein the first heavy chain variable region comprises a complementarity determining region 1 of the first heavy chain variable region (H1CDR1), a complementarity determining region 2 of the first heavy chain variable region (H1CDR2) and/or a complementarity determining region 3 of the first heavy chain variable region (H1CDR3); and the first light chain variable region comprises a complementarity determining region 1 of the first light chain variable region (L1CDR1), a complementarity determining region 2 of the first light chain variable region (L1CDR2) and/or a complementarity determining region 3 of the first light chain variable region (L1CDR3); and the anti-PD-L1 antibody or antigen-binding fragment thereof is an antibody or antigen-binding fragment thereof that specifically binds to PD-L1.

In some embodiments, the present disclosure provides an anti-CD47/anti-PD-L1 antibody comprising an anti-CD47 antibody or antigen-binding fragment thereof and an anti-PD-L1 antibody or antigen-binding fragment thereof, wherein the anti-CD47 antibody or antigen-binding fragment thereof comprises a first heavy chain variable region and a first light chain variable region, wherein:
(1) the first heavy chain variable region comprises H1CDR1, H1CDR2 and H1CDR3 selected from the group consisting of:
   (a1) amino acid sequences set forth in SEQ ID NOs: 1, 2 and 3;
   (a2) amino acid sequences set forth in SEQ ID NOs: 10, 2 and 11;
   (a3) amino acid sequences set forth in SEQ ID NOs: 4, 5 and 6;
   (a4) amino acid sequences set forth in SEQ ID NOs: 7, 8 and 9; and
   (a5) amino acid sequences having at least 85% sequence identity to the amino acid sequences set forth in (a1), (a2), (a3) or (a4); and
(2) the first light chain variable region comprises L1CDR1, L1CDR2 and L1CDR3 selected from the group consisting of:
   (a6) amino acid sequences set forth in SEQ ID NOs: 12, 13 and 14;
   (a7) amino acid sequences set forth in SEQ ID NOs: 15, 16 and 17;
   (a8) amino acid sequences set forth in SEQ ID NOs: 18, 19 and 20;
   (a9) amino acid sequences having at least 85% sequence identity to the amino acid sequences set forth in (a6), (a7) or (a8).

In some embodiments, the anti-CD47 antibody or antigen-binding fragment thereof of the anti-CD47/anti-PD-L1 antibody comprises:
the first heavy chain variable region comprising H1CDR1, H1CDR2 and H1CDR3 with amino acid sequences set forth in SEQ ID NOs: 1, 2 and 3, or with amino acid sequences having at least 85% sequence identity to the amino acid sequences set forth in SEQ ID NOs: 1, 2 and 3, respectively, and the first light chain variable region comprising L1CDR1, L1CDR2 and L1CDR3 with amino acid sequences set forth in SEQ ID NOs: 12, 13 and 14, or with amino acid sequences having at least 85% sequence identity to the amino acid sequences set forth in SEQ ID NOs: 12, 13 and 14, respectively;
the first heavy chain variable region comprising H1CDR1, H1CDR2 and H1CDR3 with amino acid sequences set forth in SEQ ID NOs: 10, 2 and 11, or with amino acid sequences having at least 85% sequence identity to the amino acid sequences set forth in SEQ ID NOs: 10, 2 and 11, respectively, and the first light chain variable region comprising L1CDR1, L1CDR2 and L1CDR3 with amino acid sequences set forth in SEQ ID NOs: 12, 13 and 14, or with amino acid sequences having at least 85% sequence identity to the amino acid sequences set forth in SEQ ID NOs: 12, 13 and 14, respectively;
the first heavy chain variable region comprising H1CDR1, H1CDR2 and H1CDR3 with amino acid sequences set forth in SEQ ID NOs: 4, 5 and 6, or with amino acid sequences having at least 85% sequence identity to the amino acid sequences set forth in SEQ ID NOs: 4, 5 and 6, respectively, and the first light chain variable region comprising L1CDR1, L1CDR2 and L1CDR3 with amino acid sequences set forth in SEQ ID NOs: 15, 16 and 17, or with amino acid sequences having at least 85% sequence identity to the amino acid sequences set forth in SEQ ID NOs: 15, 16 and 17, respectively; or
the first heavy chain variable region comprising H1CDR1, H1CDR2 and H1CDR3 with amino acid sequences set forth in SEQ ID NOs: 7, 8 and 9, or with amino acid sequences having at least 85% sequence identity to the amino acid sequences set forth in SEQ ID NOs: 7, 8 and 9, respectively, and the first light chain variable region comprising L1CDR1, L1CDR2 and L1CDR3 with amino acid sequences set forth in SEQ ID NOs: 18, 19 and 20, or with amino acid sequences having at least 85% sequence identity to the amino acid sequences set forth in SEQ ID NOs: 18, 19 and 20, respectively.

In some embodiments, the anti-CD47 antibody or antigen-binding fragment thereof of the anti-CD47/anti-PD-L1 antibody comprises a first heavy chain variable region and a first light chain variable region, wherein:
(1) the first heavy chain variable region comprises an amino acid sequence selected from the group consisting of:
   (b1) amino acid sequences set forth in SEQ ID NOs: 21, 22, 23 and 27,
   (b2) amino acid sequences derived from the amino acid sequences set forth in (b1) by substitution, deletion or addition of one or more amino acids and functionally identical or similar to the amino acid sequences set forth in (b1), and
   (b3) amino acid sequences having at least 80% sequence identity to the amino acid sequences set forth in (b1); and
(2) the first light chain variable region comprises an amino acid sequence selected from the group consisting of:
   (b4) amino acid sequences set forth in SEQ ID NOs: 24, 25, 26 and 28,
   (b5) amino acid sequences derived from the amino acid sequences set forth in (b4) by substitution, deletion or addition of one or more amino acids and functionally identical or similar to the amino acid sequences set forth in (b4), and
   (b6) amino acid sequences having at least 80% sequence identity to the amino acid sequences set forth in (b4).

In some embodiments, the anti-CD47 antibody or antigen-binding fragment thereof of the anti-CD47/anti-PD-L1 antibody comprises a first heavy chain variable region and a first light chain variable region, wherein:
the first heavy chain variable region comprises an amino acid sequence selected from the group consisting of: an amino acid sequence set forth in SEQ ID NO: 21, amino acid sequences derived from SEQ ID NO: 21 by substitution, deletion or addition of one or more amino acids and functionally identical to SEQ ID NO: 21, and amino acid sequences having at least 85% sequence identity to SEQ ID NO: 21; and the first light chain variable region comprises an amino acid sequence selected from the group consisting of: an amino acid sequence set forth in SEQ ID NO: 24, amino acid sequences derived from SEQ ID NO: 24 by substitution, deletion or addition of one or more amino acids and functionally identical to SEQ ID NO: 24, and amino acid sequences having at least 85% sequence identity to SEQ ID NO: 24;
the first heavy chain variable region comprises an amino acid sequence selected from the group consisting of: an amino acid sequence set forth in SEQ ID NO: 22, amino acid sequences derived from SEQ ID NO: 22 by substitution, deletion or addition of one or more amino acids and functionally identical to SEQ ID NO: 22, and amino acid sequences having at least 85% sequence identity to SEQ ID NO: 22; and the first light chain variable region comprises an amino acid sequence selected from the group consisting of: an amino acid sequence set forth in SEQ ID NO: 25, amino acid sequences derived from SEQ ID NO: 25 by substitution, deletion or addition of one or more amino acids and functionally identical to SEQ ID NO: 25, and amino acid sequences having at least 85% sequence identity to SEQ ID NO: 25;
the first heavy chain variable region comprises an amino acid sequence selected from the group consisting of: an amino acid sequence set forth in SEQ ID NO: 23, amino acid sequences derived from SEQ ID NO: 23 by substitution, deletion or addition of one or more amino acids and functionally identical to SEQ ID NO: 23, and amino acid sequences having at least 85% sequence identity to SEQ ID NO: 23; and the first light chain variable region comprises an amino acid sequence selected from the group consisting of: an amino acid sequence set forth in SEQ ID NO: 26, amino acid sequences derived from SEQ ID NO: 26 by substitution, deletion or addition of one or more amino acids and functionally identical to SEQ ID NO: 26, and amino acid sequences having at least 85% sequence identity to SEQ ID NO: 26;
the first heavy chain variable region comprises an amino acid sequence selected from the group consisting of: an amino acid sequence set forth in SEQ ID NO: 27, amino acid sequences derived from SEQ ID NO: 27 by substitution, deletion or addition of one or more amino acids and functionally identical to SEQ ID NO: 27, and amino acid sequences having at least 85% sequence identity to SEQ ID NO: 27; and the first light chain variable region comprises an amino acid sequence selected from the group consisting of: an amino acid sequence set forth in SEQ ID NO: 24, amino acid sequences derived from SEQ ID NO: 24 by substitution, deletion or addition of one or more amino acids and functionally identical to SEQ ID NO: 24, and amino acid sequences having at least 85% sequence identity to SEQ ID NO: 24; or
the first heavy chain variable region comprises an amino acid sequence selected from the group consisting of: an amino acid sequence set forth in SEQ ID NO: 23, amino acid sequences derived from SEQ ID NO: 23 by substitution, deletion or addition of one or more amino acids and functionally identical to SEQ ID NO: 23, and amino acid sequences having at least 85% sequence identity to SEQ ID NO: 23; and the first light chain variable region comprises an amino acid sequence selected from the group consisting of: an amino acid sequence set forth in SEQ ID NO: 28, amino acid sequences derived from SEQ ID NO: 28 by substitution, deletion or addition of one or more amino acids and functionally identical to SEQ ID NO: 28, and amino acid sequences having at least 85% sequence identity to SEQ ID NO: 28.

In some embodiments, the anti-CD47 antibody or antigen-binding fragment thereof of the anti-CD47/anti-PD-L1 antibody is a humanized antibody or antigen-binding fragment thereof comprising a first heavy chain variable region and a first light chain variable region, wherein:
(1) the first heavy chain variable region comprises an amino acid sequence selected from the group consisting of:
   (c1) amino acid sequences set forth in SEQ ID NOs: 29, 30 and 31,
   (c2) amino acid sequences derived from the amino acid sequences set forth in (c1) by substitution, deletion or addition of one or more amino acids and functionally identical or similar to the amino acid sequences set forth in (c1), and
   (c3) amino acid sequences having at least 80% sequence identity to the amino acid sequences set forth in (c1); and
(2) the first light chain variable region comprises an amino acid sequence selected from the group consisting of:
   (c4) amino acid sequences set forth in SEQ ID NOs: 32, 33 and 34,
   (c5) amino acid sequences derived from the amino acid sequences set forth in (c4) by substitution, deletion or addition of one or more amino acids and functionally identical or similar to the amino acid sequences set forth in (c4), and
   (c6) amino acid sequences having at least 80% sequence identity to the amino acid sequences set forth in (c4).

In some embodiments, the anti-CD47 antibody or antigen-binding fragment thereof of the anti-CD47/anti-PD-L1 antibody is a humanized antibody or antigen-binding fragment thereof comprising a first heavy chain variable region and a first light chain variable region, wherein:
the first heavy chain variable region comprises an amino acid sequence selected from the group consisting of: an amino acid sequence set forth in SEQ ID NO: 29, amino acid sequences derived from SEQ ID NO: 29 by substitution, deletion or addition of one or more amino acids and functionally identical to SEQ ID NO: 29, and amino acid sequences having at least 85% sequence identity to SEQ ID NO: 29; and the first light chain variable region comprises an amino acid sequence selected from the group consisting of: an amino acid sequence set forth in SEQ ID NO: 32, amino acid sequences derived from SEQ ID NO: 32 by substitution, deletion or addition of one or more amino acids and functionally identical to SEQ ID NO: 32, and amino acid sequences having at least 85% sequence identity to SEQ ID NO: 32;
the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences having at least 85% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences having at least 85% sequence identity to SEQ ID NO: 33; or
the first heavy chain variable region comprises an amino acid sequence selected from the group consisting of: an amino acid sequence set forth in SEQ ID NO: 31, amino acid sequences derived from SEQ ID NO: 31 by substitution, deletion or addition of one or more amino acids and functionally identical to SEQ ID NO: 31, and amino acid sequences having at least 85% sequence identity to SEQ ID NO: 31; and the first light chain variable region comprises an amino acid sequence selected from the group consisting of: an amino acid sequence set forth in SEQ ID NO: 34, amino acid sequences derived from SEQ ID NO: 34 by substitution, deletion or addition of one or more amino acids and functionally identical to SEQ ID NO: 34, and amino acid sequences having at least 85% sequence identity to SEQ ID NO: 34.

In some embodiments, the present disclosure provides an anti-CD47/anti-PD-L1 antibody comprising an anti-CD47 antibody or antigen-binding fragment thereof and an anti-PD-L1 antibody or antigen-binding fragment thereof, wherein:
the anti-CD47 antibody or antigen-binding fragment thereof comprises a first heavy chain variable region and a first light chain variable region, wherein:
(1) the first heavy chain variable region comprises H1CDR1, H1CDR2 and H1CDR3 with amino acid sequences set forth in SEQ ID NOs: 4, 5 and 6, or with amino acid sequences having at least 85% sequence identity to the amino acid sequences set forth in SEQ ID NOs: 4, 5 and 6, respectively; and
(2) the first light chain variable region comprises L1CDR1, L1CDR2 and L1CDR3 with amino acid sequences set forth in SEQ ID NOs: 15, 16 and 17, or with amino acid sequences having at least 85% sequence identity to the amino acid sequences set forth in SEQ ID NOs: 15, 16 and 17, respectively.

In some preferred embodiments, the present disclosure provides an anti-CD47/anti-PD-L1 antibody comprising an anti-CD47 antibody or antigen-binding fragment thereof and an anti-PD-L1 antibody or antigen-binding fragment thereof, wherein the anti-PD-L1 antibody or antigen-binding fragment thereof comprises a second heavy chain variable region and/or a second light chain variable region, wherein the second heavy chain variable region comprises a complementarity determining region 1 of the second heavy chain variable region (H2CDR1), a complementarity determining region 2 of the second heavy chain variable region (H2CDR2) and/or a complementarity determining region 3 of the second heavy chain variable region (H2CDR3); and the second light chain variable region comprises a complementarity determining region 1 of the second light chain variable region (L2CDR1), a complementarity determining region 2 of the second light chain variable region (L2CDR2) and/or a complementarity determining region 3 of the second light chain variable region (L2CDR3).

Further preferably, in some embodiments, the present disclosure provides an anti-CD47/anti-PD-L1 antibody comprising an anti-CD47 antibody or antigen-binding fragment thereof and an anti-PD-L1 antibody or antigen-binding fragment thereof, wherein the anti-CD47 antibody or antigen-binding fragment thereof is as defined in the above embodiments, and the anti-PD-L1 antibody or antigen-binding fragment thereof comprises a second heavy chain variable region and a second light chain variable region, wherein:
(1) the second heavy chain variable region comprises H2CDR1, H2CDR2 and H2CDR3 selected from the group consisting of:
   (A1) amino acid sequences set forth in SEQ ID NOs: 75, 76 and 77;
   (A2) amino acid sequences set forth in SEQ ID NOs: 81, 82 and 83;
   (A3) amino acid sequences set forth in SEQ ID NOs: 87, 88 and 89;
   (A4) amino acid sequences set forth in SEQ ID NOs: 93, 94 and 95; and
   (A5) amino acid sequences having at least 85% sequence identity to the amino acid sequences set forth in (A1), (A2), (A3) or (A4); and
(2) the second light chain variable region comprises L2CDR1, L2CDR2 and L2CDR3 selected from the group consisting of:
   (A6) amino acid sequences set forth in SEQ ID NOs: 78, 79 and 80;
   (A7) amino acid sequences set forth in SEQ ID NOs: 84, 85 and 86;
   (A8) amino acid sequences set forth in SEQ ID NOs: 90, 91 and 92;
   (A9) amino acid sequences set forth in SEQ ID NOs: 96, 97 and 98; and
   (A10) amino acid sequences having at least 85% sequence identity to the amino acid sequences set forth in (A6), (A7), (A8) or (A9).

In some specific embodiments, the present disclosure provides an anti-CD47/anti-PD-L1 antibody comprising an anti-CD47 antibody or antigen-binding fragment thereof and an anti-PD-L1 antibody or antigen-binding fragment thereof, wherein:
the anti-CD47 antibody or antigen-binding fragment thereof comprises a first heavy chain variable region and a first light chain variable region, wherein:
   (1) the first heavy chain variable region comprises H1CDR1, H1CDR2 and H1CDR3 comprising amino acid sequences set forth in SEQ ID NOs: 4, 5 and 6, or amino acid sequences having at least 85% sequence identity to the amino acid sequences set forth in SEQ ID NOs: 4, 5 and 6, respectively; and
   (2) the first light chain variable region comprises L1CDR1, L1CDR2 and L1CDR3 comprising amino acid sequences set forth in SEQ ID NOs: 15, 16 and 17, or amino acid sequences having at least 85% sequence identity to the amino acid sequences set forth in SEQ ID NOs: 15, 16 and 17, respectively; and
the anti-PD-L1 antibody or antigen-binding fragment thereof comprises a second heavy chain variable region and a second light chain variable region, wherein:
   (1) the second heavy chain variable region comprises H2CDR1, H2CDR2 and H2CDR3 selected from the group consisting of:
      (A1) amino acid sequences set forth in SEQ ID NOs: 75, 76 and 77;
      (A2) amino acid sequences set forth in SEQ ID NOs: 81, 82 and 83;
      (A3) amino acid sequences set forth in SEQ ID NOs: 87, 88 and 89;
      (A4) amino acid sequences set forth in SEQ ID NOs: 93, 94 and 95; and
      (A5) amino acid sequences having at least 85% sequence identity to the amino acid sequences set forth in (A1), (A2), (A3) or (A4); and
(2) the second light chain variable region comprises L2CDR1, L2CDR2 and L2CDR3 selected from the group consisting of:
   (A6) amino acid sequences set forth in SEQ ID NOs: 78, 79 and 80;
   (A7) amino acid sequences set forth in SEQ ID NOs: 84, 85 and 86;
   (A8) amino acid sequences set forth in SEQ ID NOs: 90, 91 and 92;
   (A9) amino acid sequences set forth in SEQ ID NOs: 96, 97 and 98; and
   (A10) amino acid sequences having at least 85% sequence identity to the amino acid sequences set forth in (A6), (A7), (A8) or (A9).

In a specific embodiment, the present disclosure provides an anti-CD47/anti-PD-L1 antibody comprising an anti-CD47 antibody or antigen-binding fragment thereof and an anti-PD-L1 antibody or antigen-binding fragment thereof. The first heavy chain variable region of the anti-CD47 antibody or antigen-binding fragment thereof comprises H1CDR1, H1CDR2 and H1CDR3 comprising amino acid sequences set forth in SEQ ID NOs: 4, 5 and 6, or amino acid sequences having at least 85% sequence identity to the amino acid sequences set forth in SEQ ID NOs: 4, 5 and 6, respectively, and the first light chain variable region comprises L1CDR1, L1CDR2 and L1CDR3 comprising amino acid sequences set forth in SEQ ID NOs: 15, 16 and 17, or amino acid sequences having at least 85% sequence identity to the amino acid sequences set forth in SEQ ID NOs: 15, 16 and 17, respectively. The second heavy chain variable region of the anti-PD-L1 antibody or antigen-binding fragment thereof comprises H2CDR1, H2CDR2 and H2CDR3 comprising amino acid sequences set forth in SEQ ID NOs: 75, 76 and 77, or amino acid sequences having at least 85% sequence identity to the amino acid sequences set forth in SEQ ID NOs: 75, 76 and 77, respectively, and the second light chain variable region comprises L2CDR1, L2CDR2 and L2CDR3 comprising amino acid sequences set forth in SEQ ID NOs: 78, 79 and 80, or amino acid sequences having at least 85% sequence identity to the amino acid sequences set forth in SEQ ID NOs: 78, 79 and 80, respectively.

In a specific embodiment, the present disclosure provides an anti-CD47/anti-PD-L1 antibody comprising an anti-CD47 antibody or antigen-binding fragment thereof and an anti-PD-L1 antibody or antigen-binding fragment thereof. The first heavy chain variable region of the anti-CD47 antibody or antigen-binding fragment thereof comprises H1CDR1, H1CDR2 and H1CDR3 comprising amino acid sequences set forth in SEQ ID NOs: 4, 5 and 6, or amino acid sequences having at least 85% sequence identity to the amino acid sequences set forth in SEQ ID NOs: 4, 5 and 6, respectively, and the first light chain variable region comprises L1CDR1, L1CDR2 and L1CDR3 comprising amino acid sequences set forth in SEQ ID NOs: 15, 16 and 17, or amino acid sequences having at least 85% sequence identity to the amino acid sequences set forth in SEQ ID NOs: 15, 16 and 17, respectively. The second heavy chain variable region of the anti-PD-L1 antibody or antigen-binding fragment thereof comprises H2CDR1, H2CDR2 and H2CDR3 comprising amino acid sequences set forth in SEQ ID NOs: 81, 82 and 83, or amino acid sequences having at least 85% sequence identity to the amino acid sequences set forth in SEQ ID NOs: 81, 82 and 83, respectively, and the second light chain variable region comprises L2CDR1, L2CDR2 and L2CDR3 comprising amino acid sequences set forth in SEQ ID NOs: 84, 85 and 86, or amino acid sequences having at least 85% sequence identity to the amino acid sequences set forth in SEQ ID NOs: 84, 85 and 86, respectively.

In a specific embodiment, the present disclosure provides an anti-CD47/anti-PD-L1 antibody comprising an anti-CD47 antibody or antigen-binding fragment thereof and an anti-PD-L1 antibody or antigen-binding fragment thereof. The first heavy chain variable region of the anti-CD47 antibody or antigen-binding fragment thereof comprises H1CDR1, H1CDR2 and H1CDR3 comprising amino acid sequences set forth in SEQ ID NOs: 4, 5 and 6, or amino acid sequences having at least 85% sequence identity to the amino acid sequences set forth in SEQ ID NOs: 4, 5 and 6, respectively, and the first light chain variable region comprises L1CDR1, L1CDR2 and L1CDR3 comprising amino acid sequences set forth in SEQ ID NOs: 15, 16 and 17, or amino acid sequences having at least 85% sequence identity to the amino acid sequences set forth in SEQ ID NOs: 15, 16 and 17, respectively. The second heavy chain variable region of the anti-PD-L1 antibody or antigen-binding fragment thereof comprises H2CDR1, H2CDR2 and H2CDR3 comprising amino acid sequences set forth in SEQ ID NOs: 87, 88 and 89, or amino acid sequences having at least 85% sequence identity to the amino acid sequences set forth in SEQ ID NOs: 87, 88 and 89, respectively, and the second light chain variable region comprises L2CDR1, L2CDR2 and L2CDR3 comprising amino acid sequences set forth in SEQ ID NOs: 90, 91 and 92, or amino acid sequences having at least 85% sequence identity to the amino acid sequences set forth in SEQ ID NOs: 90, 91 and 92, respectively.

In a specific embodiment, the present disclosure provides an anti-CD47/anti-PD-L1 antibody comprising an anti-CD47 antibody or antigen-binding fragment thereof and an anti-PD-L1 antibody or antigen-binding fragment thereof. The first heavy chain variable region of the anti-CD47 antibody or antigen-binding fragment thereof comprises H1CDR1, H1CDR2 and H1CDR3 comprising amino acid sequences set forth in SEQ ID NOs: 4, 5 and 6, or amino acid sequences having at least 85% sequence identity to the amino acid sequences set forth in SEQ ID NOs: 4, 5 and 6, respectively, and the first light chain variable region comprises L1CDR1, L1CDR2 and L1CDR3 comprising amino acid sequences set forth in SEQ ID NOs: 15, 16 and 17, or amino acid sequences having at least 85% sequence identity to the amino acid sequences set forth in SEQ ID NOs: 15, 16 and 17, respectively. The second heavy chain variable region of the anti-PD-L1 antibody or antigen-binding fragment thereof comprises H2CDR1, H2CDR2 and H2CDR3 comprising amino acid sequences set forth in SEQ ID NOs: 93, 94 and 95, or amino acid sequences having at least 85% sequence identity to the amino acid sequences set forth in SEQ ID NOs: 93, 94 and 95, respectively, and the second light chain variable region comprises L2CDR1, L2CDR2 and L2CDR3 comprising amino acid sequences set forth in SEQ ID NOs: 96, 97 and 98, or amino acid sequences having at least 85% sequence identity to the amino acid sequences set forth in SEQ ID NOs: 96, 97 and 98, respectively.

In some specific embodiments, for the anti-CD47/anti-PD-L1 antibody according to the present disclosure, the anti-CD47 antibody or antigen-binding fragment thereof and the anti-PD-L1 antibody or antigen-binding fragment thereof are each independently a murine-derived antibody, a chimeric antibody, a humanized antibody or a fully human antibody.

In some specific embodiments, the present disclosure provides an anti-CD47/anti-PD-L1 antibody, wherein the anti-CD47 antibody or antigen-binding fragment thereof comprises a first heavy chain variable region and a first light chain variable region, wherein:
(1) the first heavy chain variable region comprises an amino acid sequence selected from the group consisting of:
   (b1) amino acid sequences set forth in SEQ ID NO: 22 and SEQ ID NO: 30,
   (b2) amino acid sequences derived from the amino acid sequences set forth in (b1) by substitution, deletion or addition of one or more amino acids and functionally identical or similar to the amino acid sequences set forth in (b1), and
   (b3) amino acid sequences having at least 80% sequence identity to the amino acid sequences set forth in (b1); and
(2) the first light chain variable region comprises an amino acid sequence selected from the group consisting of:
   (b4) amino acid sequences set forth in SEQ ID NO: 25 and SEQ ID NO: 33,
   (b5) amino acid sequences derived from the amino acid sequences set forth in (b4) by substitution, deletion or addition of one or more amino acids and functionally identical or similar to the amino acid sequences set forth in (b4), and
   (b6) amino acid sequences having at least 80% sequence identity to the amino acid sequences set forth in (b4); and
   the anti-PD-L1 antibody or antigen-binding fragment thereof comprises a second heavy chain variable region and a second light chain variable region, wherein:
      (1) the second heavy chain variable region comprises an amino acid sequence selected from the group consisting of:
         (B1) amino acid sequences set forth in SEQ ID NOs: 99, 100, 101, 102, 110, 111, 112, 113, 114, 119, 120, 121, 122 and 123,
         (B2) amino acid sequences derived from the amino acid sequences set forth in (B1) by substitution, deletion or addition of one or more amino acids and functionally identical or similar to the amino acid sequences set forth in (B1), and
         (B3) amino acid sequences having at least 80% sequence identity to the amino acid sequences set forth in (B1); and
      (2) the second light chain variable region comprises an amino acid sequence selected from the group consisting of:
         (B4) amino acid sequences set forth in SEQ ID NOs: 103, 104, 105, 106, 115, 116, 117, 118, 124, 125 and 126,
         (B5) amino acid sequences derived from the amino acid sequences set forth in (B4) by substitution, deletion or addition of one or more amino acids and functionally identical or similar to the amino acid sequences set forth in (B4), and
         (B6) amino acid sequences having at least 80% sequence identity to the amino acid sequences set forth in (B4).

In some specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 22, amino acid sequences derived from SEQ ID NO: 22 by substitution, deletion or addition of one or more amino acids and functionally identical to SEQ ID NO: 22, and amino acid sequences having at least 85% sequence identity to SEQ ID NO: 22; and the first light chain variable region comprises an amino acid sequence selected from the group consisting of: an amino acid sequence set forth in SEQ ID NO: 25, amino acid sequences derived from SEQ ID NO: 25 by substitution, deletion or addition of one or more amino acids and functionally identical to SEQ ID NO: 25, and amino acid sequences having at least 85% sequence identity to SEQ ID NO: 25.

In some specific embodiments, the present disclosure provides an anti-CD47/anti-PD-L1 antibody, wherein the first heavy chain variable region has an amino acid sequence selected from the group consisting of: an amino acid sequence set forth in SEQ ID NO: 22, amino acid sequences derived from SEQ ID NO: 22 by substitution, deletion or addition of one or more amino acids and functionally identical to SEQ ID NO: 22, and amino acid sequences comprising H1CDR1, H1CDR2 and H1CDR3 set forth in SEQ ID NOs: 4, 5 and 6 and having at least 85% sequence identity to SEQ ID NO: 22; and the first light chain variable region comprises an amino acid sequence selected from the group consisting of: an amino acid sequence set forth in SEQ ID NO: 25, amino acid sequences derived from SEQ ID NO: 25 by substitution, deletion or addition of one or more amino acids and functionally identical to SEQ ID NO: 25, and amino acid sequences comprising L1CDR1, L1CDR2 and L1CDR3 set forth in SEQ ID NOs: 15, 16 and 17 and having at least 85% sequence identity to SEQ ID NO: 25.

In some specific embodiments, for the anti-CD47/anti-PD-L1 antibody according to the present disclosure, the anti-CD47 antibody or the anti-PD-L1 antibody may be a murine-derived antibody, which further comprises a heavy chain constant region of murine-derived IgG1, IgG2, IgG3, IgG4, or a variant thereof, and a light chain constant region of murine-derived kappa chain or a variant thereof.

In some preferred embodiments, for the anti-CD47/anti-PD-L1 antibody according to the present disclosure, the murine-derived anti-CD47 antibody further comprises a heavy chain constant region of murine-derived IgG1, IgG2, or a variant thereof, and a light chain constant region of murine-derived kappa chain or a variant thereof.

In some specific embodiments, the present disclosure provides an anti-CD47/anti-PD-L1 antibody, wherein the anti-CD47 antibody or antigen-binding fragment thereof comprises a first heavy chain variable region and a first light chain variable region, wherein:
(1) the first heavy chain variable region comprises an amino acid sequence selected from the group consisting of:
   (c1) an amino acid sequence set forth in SEQ ID NO: 30,
   (c2) amino acid sequences derived from the amino acid sequences set forth in (c1) by substitution, deletion or addition of one or more amino acids and functionally identical or similar to the amino acid sequences set forth in (c1), and
   (c3) amino acid sequences having at least 80% sequence identity to the amino acid sequences set forth in (c1); and
(2) the first light chain variable region comprises an amino acid sequence selected from the group consisting of:
   (c4) an amino acid sequence set forth in SEQ ID NO: 33,
   (c5) amino acid sequences derived from the amino acid sequences set forth in (c4) by substitution, deletion or addition of one or more amino acids and functionally identical or similar to the amino acid sequences set forth in (c4), and
   (c6) amino acid sequences having at least 80% sequence identity to the amino acid sequences set forth in (c4); and
   the anti-PD-L1 antibody or antigen-binding fragment thereof comprises a second heavy chain variable region and a second light chain variable region, wherein:
      (1) the second heavy chain variable region comprises an amino acid sequence selected from the group consisting of:
         (C1) amino acid sequences set forth in SEQ ID NOs: 110, 111, 112, 113, 114, 119, 120, 121, 122 and 123,
         (C2) amino acid sequences derived from the amino acid sequences set forth in (C1) by substitution, deletion or addition of one or more amino acids and functionally identical or similar to the amino acid sequences set forth in (C1), and
         (C3) amino acid sequences having at least 80% sequence identity to the amino acid sequences set forth in (C1); and
      (2) the second light chain variable region comprises an amino acid sequence selected from the group consisting of:
         (C4) amino acid sequences set forth in SEQ ID NOs: 115, 116, 117, 118, 124, 125 and 126,
         (C5) amino acid sequences derived from the amino acid sequences set forth in (C4) by substitution, deletion or addition of one or more amino acids and functionally identical or similar to the amino acid sequences set forth in (C4), and
         (C6) amino acid sequences having at least 80% sequence identity to the amino acid sequences set forth in (C4).

In some specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences having at least 85% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences having at least 85% sequence identity to SEQ ID NO: 33; and the second heavy chain variable region comprises an amino acid sequence selected from the group consisting of: amino acid sequences set forth in SEQ ID NOs: 110, 111, 112, 113 and 114, amino acid sequences derived from SEQ ID NO: 110, 111, 112, 113 or 114 by substitution, deletion or addition of one or more amino acids and functionally identical to SEQ ID NO: 110, 111, 112, 113 or 114, and amino acid sequences having at least 85% sequence identity to SEQ ID NO: 110, 111, 112, 113 or 114; and the second light chain variable region comprises an amino acid sequence selected from the group consisting of: amino acid sequences set forth in SEQ ID NOs: 115, 116, 117 and 118, amino acid sequences derived from SEQ ID NO: 115, 116, 117 or 118 by substitution, deletion or addition of one or more amino acids and functionally identical to SEQ ID NO: 115, 116, 117 or 118, and amino acid sequences having at least 85% sequence identity to SEQ ID NO: 115, 116, 117 or 118.

In some specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences having at least 85% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences having at least 85% sequence identity to SEQ ID NO: 33; and the second heavy chain variable region comprises an amino acid sequence selected from the group consisting of: amino acid sequences set forth in SEQ ID NOs: 119, 120, 121, 122 and 123, amino acid sequences derived from SEQ ID NO: 119, 120, 121, 122 or 123 by substitution, deletion or addition of one or more amino acids and functionally identical to SEQ ID NO: 119, 120, 121, 122 or 123, and amino acid sequences having at least 85% sequence identity to SEQ ID NO: 119, 120, 121, 122 or 123; and the second light chain variable region comprises an amino acid sequence selected from the group consisting of: amino acid sequences set forth in SEQ ID NOs: 124, 125 and 126, amino acid sequences derived from SEQ ID NO: 124, 125 or 126 by substitution, deletion or addition of one or more amino acids and functionally identical to SEQ ID NO: 124, 125 or 126, and amino acid sequences having at least 85% sequence identity to SEQ ID NO: 124, 125 or 126.

In some specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences comprising H1CDR1, H1CDR2 and H1CDR3 set forth in SEQ ID NOs: 4, 5 and 6 and having at least 85% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences comprising L1CDR1, L1CDR2 and L1CDR3 set forth in SEQ ID NOs: 15, 16 and 17 and having at least 85% sequence identity to SEQ ID NO: 33; and the second heavy chain variable region comprises an amino acid sequence selected from the group consisting of: amino acid sequences set forth in SEQ ID NOs: 110, 111, 112, 113 and 114, amino acid sequences derived from SEQ ID NO: 110, 111, 112, 113 or 114 by substitution, deletion or addition of one or more amino acids and functionally identical to SEQ ID NO: 110, 111, 112, 113 or 114, and amino acid sequences comprising H2CDR1, H2CDR2 and H2CDR3 set forth in SEQ ID NOs: 75, 76 and 77 and having at least 85% sequence identity to SEQ ID NO: 110, 111, 112, 113 or 114; and the second light chain variable region comprises an amino acid sequence selected from the group consisting of: amino acid sequences set forth in SEQ ID NOs: 115, 116, 117 and 118, amino acid sequences derived from SEQ ID NO: 115, 116, 117 or 118 by substitution, deletion or addition of one or more amino acids and functionally identical to SEQ ID NO: 115, 116, 117 or 118, and amino acid sequences comprising L2CDR1, L2CDR2 and L2CDR3 set forth in SEQ ID NOs: 78, 79 and 80 and having at least 85% sequence identity to SEQ ID NO: 115, 116, 117 or 118.

In some specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences comprising H1CDR1, H1CDR2 and H1CDR3 set forth in SEQ ID NOs: 4, 5 and 6 and having at least 85% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences comprising L1CDR1, L1CDR2 and L1CDR3 set forth in SEQ ID NOs: 15, 16 and 17 and having at least 85% sequence identity to SEQ ID NO: 33; and the second heavy chain variable region comprises an amino acid sequence selected from the group consisting of: amino acid sequences set forth in SEQ ID NOs: 110, 111, 112, 113 and 114, amino acid sequences derived from SEQ ID NO: 110, 111, 112, 113 or 114 by substitution, deletion or addition of one or more amino acids and functionally identical to SEQ ID NO: 110, 111, 112, 113 or 114, and amino acid sequences comprising H2CDR1, H2CDR2 and H2CDR3 set forth in SEQ ID NOs: 87, 88 and 89 and having at least 85% sequence identity to SEQ ID NO: 110, 111, 112, 113 or 114; and the second light chain variable region comprises an amino acid sequence selected from the group consisting of: amino acid sequences set forth in SEQ ID NOs: 115, 116, 117 and 118, amino acid sequences derived from SEQ ID NO: 115, 116, 117 or 118 by substitution, deletion or addition of one or more amino acids and functionally identical to SEQ ID NO: 115, 116, 117 or 118, and amino acid sequences comprising L2CDR1, L2CDR2 and L2CDR3 set forth in SEQ ID NOs: 90, 91 and 92 and having at least 85% sequence identity to SEQ ID NO: 115, 116, 117 or 118.

In some specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences comprising H1CDR1, H1CDR2 and H1CDR3 set forth in SEQ ID NOs: 4, 5 and 6 and having at least 85%, at least 90%, at least 95%, or at least 98% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences comprising L1CDR1, L1CDR2 and L1CDR3 set forth in SEQ ID NOs: 15, 16 and 17 and having at least 85%, at least 90%, at least 95%, or at least 98% sequence identity to SEQ ID NO: 33; and the second heavy chain variable region comprises an amino acid sequence selected from the group consisting of: an amino acid sequence set forth in SEQ ID NO: 112, amino acid sequences derived from SEQ ID NO: 112 by substitution, deletion or addition of one or more amino acids and functionally identical to SEQ ID NO: 112, and amino acid sequences comprising H2CDR1, H2CDR2 and H2CDR3 set forth in SEQ ID NOs: 75, 76 and 77 and having at least 85%, at least 90%, at least 95%, or at least 98% sequence identity to SEQ ID NO: 112; and the second light chain variable region comprises an amino acid sequence selected from the group consisting of: an amino acid sequence set forth in SEQ ID NO: 116, amino acid sequences derived from SEQ ID NO: 116 by substitution, deletion or addition of one or more amino acids and functionally identical to SEQ ID NO: 116, and amino acid sequences comprising L2CDR1, L2CDR2 and L2CDR3 set forth in SEQ ID NOs: 78, 79 and 80 and having at least 85%, at least 90%, at least 95%, or at least 98% sequence identity to SEQ ID NO: 116.

In other specific embodiments, the present disclosure provides an anti-CD47/anti-PD-L1 antibody, wherein the first heavy chain variable region comprises an amino acid sequence selected from the group consisting of: an amino acid sequence set forth in SEQ ID NO: 30, amino acid sequences derived from SEQ ID NO: 30 by substitution, deletion or addition of one or more amino acids and functionally identical to SEQ ID NO: 30, and amino acid sequences comprising H1CDR1, H1CDR2 and H1CDR3 set forth in SEQ ID NOs: 4, 5 and 6 and having at least 85%, at least 90%, at least 95%, or at least 98% sequence identity to SEQ ID NO: 30; and the first light chain variable region comprises an amino acid sequence selected from the group consisting of: an amino acid sequence set forth in SEQ ID NO: 33, amino acid sequences derived from SEQ ID NO: 33 by substitution, deletion or addition of one or more amino acids and functionally identical to SEQ ID NO: 33, and amino acid sequences comprising L1CDR1, L1CDR2 and L1CDR3 set forth in SEQ ID NOs: 15, 16 and 17 and having at least 85%, at least 90%, at least 95%, or at least 98% sequence identity to SEQ ID NO: 33; and the second heavy chain variable region comprises an amino acid sequence selected from the group consisting of: an amino acid sequence set forth in SEQ ID NO: 123, amino acid sequences derived from SEQ ID NO: 123 by substitution, deletion or addition of one or more amino acids and functionally identical to SEQ ID NO: 123, and amino acid sequences comprising H2CDR1, H2CDR2 and H2CDR3 set forth in SEQ ID NOs: 87, 88 and 89 and having at least 85%, at least 90%, at least 95%, or at least 98% sequence identity to SEQ ID NO: 123; and the second light chain variable region comprises an amino acid sequence selected from the group consisting of: an amino acid sequence set forth in SEQ ID NO: 126, amino acid sequences derived from SEQ ID NO: 126 by substitution, deletion or addition of one or more amino acids and functionally identical to SEQ ID NO: 126, and amino acid sequences comprising L2CDR1, L2CDR2 and L2CDR3 set forth in SEQ ID NOs: 90, 91 and 92 and having at least 85%, at least 90%, at least 95%, or at least 98% sequence identity to SEQ ID NO: 126.

In some embodiments, the present disclosure provides an anti-CD47/anti-PD-L1 humanized antibody, wherein the heavy chain comprises a heavy chain constant region of humanized IgG1, IgG2, IgG3, IgG4, or a variant thereof, and the light chain comprises a light chain constant region of humanized kappa chain, lambda chain, or a variant thereof.

In a preferred embodiment of the present disclosure, the murine-derived anti-CD47/anti-PD-L1 antibody may further comprise a light chain constant region of murine-derived kappa chain, lambda chain, or a variant thereof, and/or further comprise a heavy chain constant region of murine-derived IgG1, IgG2, IgG3, IgG4, or a variant thereof.

In a specific embodiment of the present disclosure, for the anti-CD47/anti-PD-L1 antibody according to the present disclosure, the light chain of the anti-CD47 antibody or antigen-binding fragment thereof further comprises a light chain constant region of murine-derived kappa chain, lambda chain, or mutant sequences thereof. The heavy chain of the anti-CD47 antibody or antigen-binding fragment thereof further comprises a heavy chain constant region of murine-derived IgG1, IgG2, IgG3, IgG4, or mutant sequences thereof, preferably a heavy chain constant region of humanized IgG1, IgG2 or IgG4.

In some specific embodiments, for the anti-CD47/anti-PD-L1 antibody according to the present disclosure, the anti-CD47 humanized antibody or antigen-binding fragment thereof further comprises a heavy chain constant region of humanized IgG1, IgG2, IgG3, IgG4, or a variant thereof, and a light chain constant region of humanized kappa chain, lambda chain, or a variant thereof. In some preferred embodiments, the anti-CD47 humanized antibody or antigen-binding fragment thereof of the present disclosure further comprises a heavy chain constant region of humanized IgG1, IgG2, IgG4, or a variant thereof, and a light chain constant region of humanized kappa chain or a variant thereof.

In a preferred embodiment of the present disclosure, for the anti-CD47/anti-PD-L1 antibody according to the present disclosure, the antibody heavy chain of the anti-PD-L1 antibody or antigen-binding fragment thereof further comprises a heavy chain constant region of murine-derived IgG1, IgG2, IgG3, IgG4, or mutant sequences thereof, preferably a heavy chain constant region of humanized IgG or mutant sequences thereof; and the antibody light chain of the anti-PD-L1 antibody or antigen-binding fragment thereof further comprises a light chain constant region of murine-derived kappa chain, lambda chain, or mutant sequences thereof.

In some specific embodiments, for the anti-CD47/anti-PD-L1 antibody according to the present disclosure, the anti-PD-L1 humanized antibody or antigen-binding fragment thereof further comprises a heavy chain constant region of humanized IgG1, IgG2, IgG3, IgG4, or a variant thereof, and a light chain constant region of humanized kappa chain, lambda chain, or a variant thereof. In some preferred embodiments, the anti-CD47 humanized antibody or antigen-binding fragment thereof of the present disclosure further comprises a heavy chain constant region of humanized IgG4 or a variant thereof, and a light chain constant region of humanized kappa chain or a variant thereof.

In some embodiments, the present disclosure provides an anti-CD47/anti-PD-L1 antibody, wherein the anti-CD47 antibody or antigen-binding fragment thereof and the anti-PD-L1 antibody or antigen-binding fragment thereof are each independently Fab, Fv, sFv or F(ab)₂. In a specific embodiment, the anti-CD47/anti-PD-L1 antibody provided by the present disclosure is scF(ab)₂.

Preferably, the anti-CD47/anti-PD-L1 antibody in the above embodiments of the present disclosure is an anti-CD47/anti-PD-L1 bispecific antibody. In some embodiments, the bispecific antibody is a human antibody or a humanized antibody. In some embodiments, one of the binding sites is for CD47 and the other is for any other antigen. In some embodiments, one of the binding sites is for CD47 and the other is for PD-L1. In some embodiments, the bispecific antibody binds to two different epitopes of CD47. The bispecific antibodies can also be used to localize a cytotoxic agent to cells expressing CD47. These antibodies possess a CD47 binding arm and a binding arm of a cytotoxic agent such as saporin, anti-interferon-a, vinca alkaloids, ricin A chain, methotrexate or radioisotope haptens. The bispecific antibody of the present disclosure may be prepared as a full-length antibody or antibody fragments (e.g., F(ab')₂ bispecific antibody).

Methods for preparing bispecific antibodies are known in the art. Traditionally, the recombination and preparation of bispecific antibodies are based on the co-expression of two heavy chain-light chain pairs of immunoglobulin, where the two heavy chains have different specificities (Millstein and Cuello, Nature 305: 537 (1983)). Due to the random assortment of immunoglobulin heavy and light chains, the quadromas may produce a mixture of 10 different antibody molecules, only one of which has the correct bispecific structure. Purification of this correct molecule is usually carried out by an affinity chromatography, which is rather cumbersome and product yield is low. Similar methods are disclosed in WO93/08829 and Traunecker et al., EMBOJ. 10:3655 (1991).

According to a different method, an antibody variable region with the desired binding specificities (antibody-antigen binding sites) is fused to an immunoglobulin constant region. In some embodiments, it is fused to an immunoglobulin heavy chain constant region comprising at least a portion of the hinge region, CH2 and CH3 regions. In some embodiments, the first heavy chain constant region (CHI) comprising the site necessary for binding to the light chain is present in at least a portion of the fusion. The DNA encoding the fusion fragment of the immunoglobulin heavy chain and, if desired, the immunoglobulin light chain, are inserted into separate expression vectors and co-transfected into a suitable host organism. This provides great flexibility for adjusting the mutual ratios of the three polypeptide fragments in embodiments where unequal ratios of the three polypeptide chains used in the construction provide optimal yields. However, it is possible to insert the coding sequences for two or all three polypeptide chains into one expression vector when at least two polypeptide chains are expressed in equal ratios to produce high yields or when the ratios are not of particular interest.

In one embodiment, the bispecific antibody is comprised of a hybrid immunoglobulin heavy chain with a first binding site in one arm and a hybrid immunoglobulin heavy chain-light chain pair in the other arm (providing a second binding site). Since the presence of the immunoglobulin light chain in only one half of the bispecific molecule provides a convenient separation route, this asymmetric structure facilitates separation of the desired bispecific material from the undesired immunoglobulin chain composition. This method is disclosed in WO 94/04690. For further information on the production of bispecific antibodies, reference could be made to, e.g., Suresh et al., Methods in Enzymology 121: 210 (1986).

According to another method, the interface between a pair of antibody molecules may be engineered to maximize the percentage of heterodimers recovered from recombinant cell culture. The interface comprises at least a portion of the CH3 domain of the antibody constant region. In this method, one or more small amino acids on the side chain at the interface of the first antibody molecule are replaced with big amino acids (e.g., tyrosine or tryptophan). By replacing big amino acids on the side chain with smaller amino acids (e.g., alanine or threonine), a compensatory "hole" of the same or similar size as the large side chain is created at the interface of the second antibody molecule. This provides a mechanism for heterodimers with increased yield than other undesired end products such as homodimers.

Bispecific antibodies include cross-linked or "heteroconjugated" antibodies. For example, a heteroconjugated antibody may be conjugated to avidin and the other heteroconjugated antibody may be conjugated to biotin. Heteroconjugated antibodies can be prepared using any convenient cross-linking method. Suitable crosslinking agents are well known in the art and disclosed in US Pat. No. 4,676,980, along with a number of crosslinking techniques.

The bispecific antibody of the present disclosure may be produced from antibody fragments. For example, bispecific antibodies may be prepared using chemical ligation techniques. Brennan et al., Science 229: 81 (1985) describes the proteolytic cleavage of intact antibodies to generate F(ab')₂ fragments. These fragments are decomposed in the presence of the dithiol complexing agent sodium arsenite (to stabilize adjacent dithiols and prevent the formation of intermolecular disulfide bonds). The resulting Fab' fragments were then converted into thionitrobenzoate (TNB) derivatives. One of the Fab'-TNB derivatives was then reconverted to Fab'-thiol by reduction with mercaptoethylamine and mixed with an equimolar amount of the other Fab'-TNB derivative to form a bispecific antibody.

Fab'-SH fragments may be recovered directly from E. coli and these fragments may be chemically coupled to form a bispecific antibody. Shalaby et al., J. Exp. Med. 175: 217-225 (1992) describes the generation of fully humanized bispecific antibody F(ab')₂ molecules. Each Fab' fragment is secreted separately from E. coli and subjected to targeted chemical coupling *in vitro* to form a bispecific antibody.

In some embodiments, bispecific antibody fragments of the present disclosure may be produced and isolated directly from recombinant cell culture. For example, bispecific antibodies may be generated using leucine zippers (Kostelny et al., J. Immunol. 148(5): 1547-1553 (1992)). Leucine zipper segments from Fos and Jun proteins were linked to the Fab' portions of two different antibodies by genetic fusion. Antibody homodimers are decomposed at the hinge region to form monomers, which are then reoxidized to form antibody heterodimers. This method can also be used to generate antibody homodimers. Diabody technology provides additional mechanisms for preparing bispecific antibody fragments. The bispecific antibody fragment comprises a heavy chain variable region (VH) and a light chain variable region (VL) joined by a linker that is too short to allow pairing between the two domains on the same chain. Thus, the VH and VL domains on one fragment are forced to pair with the complementary VL and VH domains on the other fragment, thereby forming two antigen binding sites. In another embodiment, bispecific antibody fragments may be constructed by using single-chain Fv (sFv) dimers.

The present disclosure encompasses multi-specific antibodies with more than two binding sites. For example, trispecific antibodies may be prepared. Multi-specific antibodies may be assimilated (and/or dissimilated) more rapidly than bivalent antibodies by cells expressing the antigens to which the antibody binds. The antibody of the present disclosure may be a multi-specific antibody (e.g., a tetravalent antibody) having three or more antigen binding sites that can be readily produced by recombinant expression of nucleic acids encoding antibody polypeptide chains. Multi-specific antibodies may contain a dimerization domain and three or more antigen binding sites. In some embodiments, the dimerization domain comprises (or consists of) an Fc region or a hinge region. In such case, the antibody will comprise an Fc region and three or more antigen binding sites amino-terminal to the Fc region. In some embodiments, the multi-specific antibody comprises (or consists of) three to about eight antigen binding sites. In some embodiments, the multi-specific antibody comprises four antigen binding sites. Multi-specific antibodies comprise at least one polypeptide chain (e.g., two polypeptide chains), wherein the polypeptide chains comprise two or more variable regions. The multi-specific antibody of the present disclosure may further comprise at least two (e.g., four) light chain variable region polypeptides. The multi-specific antibody of the present disclosure may comprise, for example, about two to about eight light chain variable region polypeptides. The light chain variable region polypeptide of the present disclosure comprises a light chain variable region, and optionally, further comprises a CL domain.

In a bispecific antibody comprising a CD47 targeting moiety and a PD-L1 targeting moiety, one of the CD47 targeting moiety and the PD-L1 targeting moiety may be a full-length antibody, and the other may be an antigen binding fragment (e.g., scFv) comprising heavy chain CDRs, light chain CDRs or a combination thereof. A full-length antibody targeting one of the CD47 and PD-L1 proteins and an antigen-binding fragment targeting the other protein can be linked directly or chemically (e.g., covalently) via a peptide linker. The antigen-binding fragment (e.g., scFv) can be linked directly or via a peptide linker to the N-terminal of the full-length antibody (e.g., the N-terminal of the light or heavy chain of the full-length antibody), the C-terminal of the full-length antibody (e.g., of the heavy chain (or Fc or CH3 domain) of the full-length antibody), or both.

In one embodiment, the bispecific antibody may comprise a full-length anti-CD47 antibody, an antigen-binding fragment of an anti-PD-L1 antibody (e.g., scFab, scFv), and a peptide linker between them. In other embodiments, the bispecific antibody may comprise a full-length anti-CD47 antibody, an antigen-binding fragment of an anti-PD-L1 antibody (e.g., scFab, scFv), and a peptide linker between them.

In one embodiment, the scFv of the bispecific antibody may comprise a heavy chain variable region and a light chain variable region in any order. For example, the scFv of the bispecific antibody may comprise a heavy chain variable region and a light chain variable region in the direction from the N-terminal to the C-terminal, and optionally a peptide linker between them, or alternatively, the scFv of the bispecific antibody may comprise a light chain variable region and a heavy chain variable region in the direction from the N-terminal to the C-terminal, and optionally a peptide linker between them.

In some embodiments, the peptide linker may comprise, for example, Gly, Asn, and/or Ser residues, and may also comprise neutral amino acids, such as Thr and/or Ala. Suitable amino acid sequences for peptide linkers may be those known in the art. Meanwhile, the length of the peptide linker may be determined differently within such limits that the function of the fusion protein is not affected. For example, the peptide linker may include a total of about 1 to about 100, about 2 to about 50, or about 5 to about 25 residues selected from the group consisting of Gly, Asn, Ser, Thr, and Ala. In one embodiment, the peptide linker may be represented as (GₘS₁)ₙ (m, l and n are independently integers from about 1 to about 10, particularly from about 2 to about 5).

In another embodiment, the PD-L1 targeting moiety and the CD47 targeting moiety may both be full-length antibodies or antigen-binding fragments comprising heavy chain CDRs, light chain CDRs, or a combination thereof.

In another embodiment, the bispecific antibody may be in the form of a heterodimer comprising a first arm and a second arm, the first arm comprising a pair of a first heavy chain and a first light chain targeting one of CD47 and PD-L1 and the second arm comprising a pair of a second heavy chain and a second light chain targeting the other.

In one embodiment, the full-length antibody may be in the form of a full-length immunoglobulin (e.g., IgG, IgM, IgA, IgE, or IgD, such as human IgG, human IgM, human IgA, human IgE, or human IgD), and the antigen-binding fragment may be selected from the group consisting of Fab, Fab', F(ab')₂, Fd, Fv, scFv, scFab, single chain antibody, sdFv and the like. For example, the full-length antibody may be in the form of a full-length human IgG (human IgG1, human IgG2, human IgG3 or human IgG4), and the antigen-binding fragment may be scFv.

For example, the antibody described herein may contain a flexible linker, or may be modified to add functional moieties (e.g., PEG, drugs, toxins, or labels).

In some specific embodiments, for the anti-CD47/anti-PD-L1 bispecific antibody according to the present disclosure, the anti-CD47 antibody or antigen-binding fragment thereof has a structure of (VL-CL)-linker-(VH)-IgG4CH, and the anti-PD-L1 antibody or antigen-binding fragment thereof has a structure of (VL-CL)-linker-(VH)-IgG4CH. In some specific embodiments, the peptide linker is in the form of (GGGGS)ₙ, wherein n is 1-12, preferably 3-10, more preferably 6-8, e.g., 6, 7 or 8 GGGGS repeats. In other specific embodiments, the IgG4CH in the (VL-CL)-linker-(VH)-IgG4CH targeting the CD47 moiety is an IgG4 CH fragment containing mutations S228P, L235E, Y349C, T366S, L368A and Y407V to form a "Hole" structure, and the IgG4CH in the (VL-CL)-linker-(VH)-IgG4CH targeting the PD-L1 moiety is an IgG4 CH fragment containing mutations S228P, L235E, T366W and S354C to form a "Knob" structure. In some specific embodiments, in the (VL-CL)-linker-(VH)-IgG4CH targeting the CD47 moiety, the VL has an amino acid sequence of SEQ ID NO: 33, the CL has an amino acid sequence of SEQ ID NO: 131, the VH has an amino acid sequence of SEQ ID NO: 30, and the IgG4 CH has an amino acid sequence of SEQ ID NO: 133; and/or in the (VL-CL)-linker-(VH)-IgG4CH targeting the PD-L1 moiety, the VL has an amino acid sequence of SEQ ID NO: 116, the CL has an amino acid sequence of SEQ ID NO: 131, the VH has an amino acid sequence of SEQ ID NO: 112, and the IgG4 CH has an amino acid sequence of SEQ ID NO: 132. In other specific embodiments, in the (VL-CL)-linker-(VH)-IgG4CH targeting the CD47 moiety, the VL has an amino acid sequence of SEQ ID NO: 33, the CL has an amino acid sequence of SEQ ID NO: 131, the VH has an amino acid sequence of SEQ ID NO: 30, and the IgG4 CH has an amino acid sequence of SEQ ID NO: 133; and/or in the (VL-CL)-linker-(VH)-IgG4CH targeting the PD-L1 moiety, the VL has an amino acid sequence of SEQ ID NO: 126, the CL has an amino acid sequence of SEQ ID NO: 131, the VH has an amino acid sequence of SEQ ID NO: 123, and the IgG4 CH has an amino acid sequence of SEQ ID NO: 132.

Another aspect of the present disclosure provides an anti-PD-L1 antibody or antigen-binding fragment thereof comprising a heavy chain variable region and a light chain variable region, wherein:
(1) the heavy chain variable region comprises CDR1, CDR2 and CDR3 selected from the group consisting of:
   (A1) amino acid sequences set forth in SEQ ID NOs: 75, 76 and 77;
   (A2) amino acid sequences set forth in SEQ ID NOs: 81, 82 and 83;
   (A3) amino acid sequences set forth in SEQ ID NOs: 87, 88 and 89;
   (A4) amino acid sequences set forth in SEQ ID NOs: 93, 94 and 95; and
   (A5) amino acid sequences having at least 85% sequence identity to the amino acid sequences set forth in (A1), (A2), (A3) or (A4); and
(2) the light chain variable region comprises CDR1, CDR2 and CDR3 selected from the group consisting of:
   (A6) amino acid sequences set forth in SEQ ID NOs: 78, 79 and 80;
   (A7) amino acid sequences set forth in SEQ ID NOs: 84, 85 and 86;
   (A8) amino acid sequences set forth in SEQ ID NOs: 90, 91 and 92;
   (A9) amino acid sequences set forth in SEQ ID NOs: 96, 97 and 98; and
   (A10) amino acid sequences having at least 85% sequence identity to the amino acid sequences set forth in (A6), (A7), (A8) or (A9).

In some embodiments, the anti-PD-L1 antibody or antigen-binding fragment thereof according to the present disclosure, wherein in the anti-PD-L1 antibody or antigen-binding fragment thereof:
the heavy chain variable region comprises CDR1, CDR2 and CDR3 comprising amino acid sequences set forth in SEQ ID NOs: 75, 76 and 77, or amino acid sequences having at least 85% sequence identity to the amino acid sequences set forth in SEQ ID NOs: 75, 76 and 77, respectively, and the light chain variable region comprises CDR1, CDR2 and CDR3 comprising amino acid sequences set forth in SEQ ID NOs: 78, 79 and 80, or amino acid sequences having at least 85% sequence identity to the amino acid sequences set forth in SEQ ID NOs: 78, 79 and 80, respectively;
the heavy chain variable region comprises CDR1, CDR2 and CDR3 comprising amino acid sequences set forth in SEQ ID NOs: 87, 88 and 89, or amino acid sequences having at least 85% sequence identity to the amino acid sequences set forth in SEQ ID NOs: 87, 88 and 89, respectively, and the light chain variable region comprises CDR1, CDR2 and CDR3 comprising amino acid sequences set forth in SEQ ID NOs: 90, 91 and 92, or amino acid sequences having at least 85% sequence identity to the amino acid sequences set forth in SEQ ID NOs: 90, 91 and 92, respectively; or
the heavy chain variable region comprises CDR1, CDR2 and CDR3 comprising amino acid sequences set forth in SEQ ID NOs: 93, 94 and 95, or amino acid sequences having at least 85% sequence identity to the amino acid sequences set forth in SEQ ID NOs: 93, 94 and 95, respectively, and the light chain variable region comprises CDR1, CDR2 and CDR3 comprising amino acid sequences set forth in SEQ ID NOs: 96, 97 and 98, or amino acid sequences having at least 85% sequence identity to the amino acid sequences set forth in SEQ ID NOs: 96, 97 and 98, respectively.

In some embodiments, the anti-PD-L1 antibody or antigen-binding fragment thereof:
(1) the heavy chain variable region comprises an amino acid sequence selected from the group consisting of:
   (B1) amino acid sequences set forth in SEQ ID NOs: 99, 100, 101, 102, 110, 111, 112, 113, 114, 119, 120, 121, 122 and 123,
   (B2) amino acid sequences derived from the amino acid sequences set forth in (B1) by substitution, deletion or addition of one or more amino acids and functionally identical or similar to the amino acid sequences set forth in (B1), and
   (B3) amino acid sequences having at least 80% sequence identity to the amino acid sequences set forth in (B1); and
(2) the light chain variable region comprises an amino acid sequence selected from the group consisting of:
   (B4) amino acid sequences set forth in SEQ ID NOs: 103, 104, 105, 106, 115, 116, 117, 118, 124, 125 and 126,
   (B5) amino acid sequences derived from the amino acid sequences set forth in (B4) by substitution, deletion or addition of one or more amino acids and functionally identical or similar to the amino acid sequences set forth in (B4), and
   (B6) amino acid sequences having at least 80% sequence identity to the amino acid sequences set forth in (B4).

In some embodiments, the anti-PD-L1 antibody or antigen-binding fragment thereof:
the heavy chain variable region comprises an amino acid sequence selected from the group consisting of: amino acid sequences set forth in SEQ ID NOs: 110, 111, 112, 113 and 114, amino acid sequences derived from SEQ ID NO: 110, 111, 112, 113 or 114 by substitution, deletion or addition of one or more amino acids and functionally identical to SEQ ID NO: 110, 111, 112, 113 or 114, and amino acid sequences having at least 85% sequence identity to SEQ ID NO: 110, 111, 112, 113 or 114; and the light chain variable region comprises an amino acid sequence selected from the group consisting of: amino acid sequences set forth in SEQ ID NOs: 115, 116, 117 and 118, amino acid sequences derived from SEQ ID NO: 115, 116, 117 or 118 by substitution, deletion or addition of one or more amino acids and functionally identical to SEQ ID NO: 115, 116, 117 or 118, and amino acid sequences having at least 85% sequence identity to SEQ ID NO: 115, 116, 117 or 118.

In some embodiments, the anti-PD-L1 antibody or antigen-binding fragment thereof:
the heavy chain variable region comprises an amino acid sequence selected from the group consisting of: amino acid sequences set forth in SEQ ID NOs: 119, 120, 121, 122 and 123, amino acid sequences derived from SEQ ID NO: 119, 120, 121, 122 or 123 by substitution, deletion or addition of one or more amino acids and functionally identical to SEQ ID NO: 119, 120, 121, 122 or 123, and amino acid sequences having at least 85% sequence identity to SEQ ID NO: 119, 120, 121, 122 or 123; and the light chain variable region comprises an amino acid sequence selected from the group consisting of: amino acid sequences set forth in SEQ ID NOs: 124, 125 and 126, amino acid sequences derived from SEQ ID NO: 124, 125 or 126 by substitution, deletion or addition of one or more amino acids and functionally identical to SEQ ID NO: 124, 125 or 126, and amino acid sequences having at least 85% sequence identity to SEQ ID NO: 124, 125 or 126.

In some embodiments, in the anti-PD-L1 antibody or antigen-binding fragment thereof according to the present disclosure, the heavy chain variable region comprises an amino acid sequence selected from the group consisting of: an amino acid sequence set forth in SEQ ID NO: 112, amino acid sequences derived from SEQ ID NO: 112 by substitution, deletion or addition of one or more amino acids and functionally identical to SEQ ID NO: 112, and amino acid sequences comprising H2CDR1, H2CDR2 and H2CDR3 set forth in SEQ ID NOs: 75, 76 and 77 and having at least 85%, at least 90%, at least 95%, or at least 98% sequence identity to SEQ ID NO: 112; and the light chain variable region comprises an amino acid sequence selected from the group consisting of: an amino acid sequence set forth in SEQ ID NO: 116, amino acid sequences derived from SEQ ID NO: 116 by substitution, deletion or addition of one or more amino acids and functionally identical to SEQ ID NO: 116, and amino acid sequences comprising L2CDR1, L2CDR2 and L2CDR3 set forth in SEQ ID NOs: 78, 79 and 80 and having at least 85%, at least 90%, at least 95%, or at least 98% sequence identity to SEQ ID NO: 116.

In some embodiments, in the anti-PD-L1 antibody or antigen-binding fragment thereof according to the present disclosure, the heavy chain variable region comprises an amino acid sequence selected from the group consisting of: an amino acid sequence set forth in SEQ ID NO: 123, amino acid sequences derived from SEQ ID NO: 123 by substitution, deletion or addition of one or more amino acids and functionally identical to SEQ ID NO: 123, and amino acid sequences comprising H2CDR1, H2CDR2 and H2CDR3 set forth in SEQ ID NOs: 87, 88 and 89 and having at least 85%, at least 90%, at least 95%, or at least 98% sequence identity to SEQ ID NO: 123; and the light chain variable region comprises an amino acid sequence selected from the group consisting of: an amino acid sequence set forth in SEQ ID NO: 126, amino acid sequences derived from SEQ ID NO: 126 by substitution, deletion or addition of one or more amino acids and functionally identical to SEQ ID NO: 126, and amino acid sequences comprising L2CDR1, L2CDR2 and L2CDR3 set forth in SEQ ID NOs: 90, 91 and 92 and having at least 85%, at least 90%, at least 95%, or at least 98% sequence identity to SEQ ID NO: 126.

In some embodiments, the anti-PD-L1 antibody or antigen-binding fragment thereof according to the present disclosure is a humanized antibody or a fully human antibody.

Another aspect of the present disclosure provides an isolated nucleic acid. In some embodiments, the isolated nucleic acid according to the present disclosure encodes an anti-CD47 /anti-PD-L1 antibody or an anti-PD-L1 antibody of the present disclosure. In some embodiments, the isolated nucleic acid according to the present disclosure encodes an anti-CD47 antibody or antigen-binding fragment thereof of the present disclosure. In other embodiments, the isolated nucleic acid according to the present disclosure encodes an anti-PD-L1 antibody or antigen-binding fragment thereof of the present disclosure.

In a specific embodiment, the nucleotide sequence encoding the first heavy chain variable region SEQ ID NO: 30 is set forth in SEQ ID NO: 36, and the nucleotide sequence encoding the first light chain variable region SEQ ID NO: 33 is set forth in SEQ ID NO: 39. In another specific embodiment, the nucleotide sequence encoding the second heavy chain variable region SEQ ID NO: 112 is set forth in SEQ ID NO: 127, and the nucleotide sequence encoding the second light chain variable region SEQ ID NO: 116 is set forth in SEQ ID NO: 129. In yet another specific embodiment, the nucleotide sequence encoding the second heavy chain variable region SEQ ID NO: 123 is set forth in SEQ ID NO: 128, and the nucleotide sequence encoding the second light chain variable region SEQ ID NO: 126 is set forth in SEQ ID NO: 130.

Another aspect of the present disclosure provides an expression vector. In some embodiments, the expression vector of the present disclosure expresses the anti-CD47/anti-PD-L1 bispecific antibody or the anti-PD-L1 antibody of the present disclosure. In some embodiments, the expression vector of the present disclosure expresses an anti-CD47 antibody or antigen-binding fragment thereof of the present disclosure. In other embodiments, the expression vector of the present disclosure expresses an anti-PD-L1 antibody or antigen-binding fragment thereof of the present disclosure. In some embodiments, the vector expressing the anti-CD47 antibody or antigen-binding fragment thereof of the present disclosure and the vector expressing the anti-PD-L1 antibody or antigen-binding fragment thereof of the present disclosure are the same expression vector. The expression vector according to the present disclosure comprises the isolated nucleic acid molecule of the present disclosure.

Another aspect of the present disclosure provides a host cell transformed with the expression vector as described above.

In some embodiments, the host cell according to the present disclosure is selected from the group consisting of prokaryotic cells and eukaryotic cells. In some embodiments, the host cell is bacteria, preferably Escherichia coli. In another preferred embodiment, the host cell is mammalian cell.

Another aspect of the present disclosure provides a method for producing the anti-CD47/anti-PD-L1 bispecific antibody, comprising expressing the antibody in the host cells and isolating the antibody from the host cells.

Another aspect of the present disclosure provides a pharmaceutical composition comprising the anti-CD47/anti-PD-L1 bispecific antibody of the present disclosure and a pharmaceutically acceptable carrier. In some embodiments, the present disclosure provides a pharmaceutical composition comprising the anti-CD47/anti-PD-L1 bispecific antibody of the present disclosure, and additional active components, such as other antibodies, targeted drugs, and the like. In some embodiments, the pharmaceutically acceptable carrier is selected from the group consisting of antioxidants, polypeptides, proteins, hydrophilic polymers, amino acids, saccharides, chelating agents, alditols, ions, and surfactants. In a specific embodiment, the pharmaceutically acceptable carrier is a buffered aqueous solution. In another specific embodiment, the pharmaceutically acceptable carrier is in the form of liposomes.

Another aspect of the present disclosure provides a chimeric antigen receptor (CAR) fusion protein comprising the anti-CD47 antibody or antigen-binding fragment thereof and/or anti-PD-L1 antibody or antigen-binding fragment thereof of the present disclosure. In some embodiments, the chimeric antigen receptor fusion protein comprises the anti-CD47 antibody or antigen-binding fragment thereof of the present disclosure, which is a single-chain variable fragment (scFv) of V_{H} and V_{L} against CD47 antigen. In other embodiments, the chimeric antigen receptor fusion protein comprises the anti-PD-L1 antibody or antigen-binding fragment thereof of the present disclosure, which is a single-chain variable fragment (scFv) of V_{H} and V_{L} against PD-L1 antigen. In other embodiments, the chimeric antigen receptor fusion protein comprises a first single-chain variable fragment (scFv) of V_{H} and V_{L} against CD47 antigen and a second single-chain variable fragment (scFv) of V_{H} and V_{L} against PD-L1 antigen. The first scFv against CD47 antigen comprises H1CDR1, H1CDR2 and H1CDR3 of the first heavy chain variable region and L1CDR1, L1CDR2 and L1CDR3 of the first light chain variable region as described in the above embodiments. The second scFv against PD-L1 antigen comprises H2CDR1, H2CDR2 and H2CDR3 of the second heavy chain variable region and L2CDR1, L2CDR2 and L2CDR3 of the second light chain variable region as described in the above embodiments.

The anti-CD47/anti-PD-L1 bispecific antibody of the present disclosure may be combined with a pharmaceutically acceptable carrier, diluent or excipient to prepare a pharmaceutical preparation suitable for oral or parenteral administration. The routes of administration include, but are not limited to oral, intradermal, intramuscular, intraperitoneal, intravenous, intracerebral, intraocular, intratracheal, subcutaneous, and intranasal routes. The preparation may be administered by any means, for example, by infusion or bolus injection, by the means of absorption through epithelium or skin mucosa (for example, oral mucosa or rectum, etc.). Administration can be systemic or local. The preparation can be prepared by methods known in the art, and contains a carrier, diluent or excipient conventionally used in the field of pharmaceutical preparations.

Another aspect of the present disclosure provides a method for treating and/or preventing a disease associated with CD47, PD-L1 or both. The method comprises administering to a subject in need thereof the anti-CD47/anti-PD-L1 bispecific antibody of the present disclosure or the pharmaceutical composition of the present disclosure.

Another aspect of the present disclosure provides use of the anti-CD47/anti-PD-L1 bispecific antibody or the anti-PD-L1 antibody of the present disclosure or the pharmaceutical composition of the present disclosure in the manufacture of a medicament for treating and/or preventing a disease associated with CD47, PD-L1 or both. In some embodiments, the disease associated with CD47, PD-L1, or both includes hematological tumor, lymphoma, breast cancer, lung cancer, gastric cancer, intestinal cancer, esophageal cancer, ovarian cancer, cervical cancer, kidney cancer, bladder cancer, pancreatic cancer, glioma and/or melanoma. The tumor may be any type of tumor that expresses PD-L1 protein, such as bladder cancer, liver cancer, colon cancer, rectal cancer, endometrial cancer, leukemia, lymphoma, pancreatic cancer, lung cancer (e.g., small cell lung cancer, non-small cell lung cancer, etc.), breast cancer, urethral cancer, head and neck cancer, gastrointestinal cancer, gastric cancer, esophageal cancer, ovarian cancer, kidney cancer, melanoma, prostate cancer, thyroid cancer, etc. The tumor may be a primary or metastatic tumor. In some embodiments, the present disclosure provides use of the above anti-CD47/anti-PD-L1 bispecific antibody or the pharmaceutical composition of the present disclosure in the manufacture of anti-tumor drugs. For example, the tumor is selected from hematological tumor, lymphoma, breast cancer, lung cancer, gastric cancer, intestinal cancer, esophageal cancer, ovarian cancer, cervical cancer, kidney cancer, bladder cancer, pancreatic cancer, glioma and melanoma.

The anti-CD47/anti-PD-L1 bispecific antibodies provided by the present disclosure have a significant anti-tumor effect and can significantly inhibit tumor growth without obvious toxicity on red blood cells. The humanized antibodies, of which the immunogenicity is greatly reduced, effectively eliminate the rejection of exogenous monoclonal antibodies by human immune system and can be used in the manufacture of medicaments for the treatment of various tumors with broad market prospects.

### DEFINITIONS

Unless otherwise defined, the meanings of scientific and technical terms used herein are those commonly understood by those skilled in the art. The nomenclature and techniques used in cell and tissue culture, molecular biology, as well as protein and oligo/polynucleotide chemistry and hybridization described herein are well known and commonly used in the art. Standard techniques are used for recombinant DNA, oligonucleotide synthesis, tissue culture and transformation (e.g. electroporation, lipofection). The enzymatic reaction and purification techniques are carried out according to the manufacturer's instructions or methods commonly used in the art or described herein. The aforementioned techniques and methods are generally used according to what's well known in the art and what's described in the multiple comprehensive and more specific documents cited and discussed in this specification. Reference could be made to, Sambrook et al., Molecular Cloning: A Laboratory Manual (2nd edition, Cold Spring Harbor Laboratory Press, Cold Spring Harbor, New York (1989)). The nomenclature as well as laboratory methods and techniques used in analytical chemistry, synthetic organic chemistry, and medical and pharmaceutical chemistry described herein are well known and commonly used in the art.

In the present disclosure, the term "at least 80% sequence identity" refers to at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity. In the present disclosure, the term "at least 85% sequence identity" refers to at least 85%, 86%, 87%, 88%, 89%, 90%, 91%, 92%, 93%, 94%, 95%, 96%, 97%, 98%, 99%, or 100% sequence identity. In some preferred embodiments, the sequence identity described in the present disclosure may be at least 90%, 91%, 92%, 93%, 94%, 95%, 96%, 97%, 98%, 99%, or 100%. Sequence comparison and determination of identity percentage between two sequences may be performed by the BLASTN/BLASTP algorithm on the website of National Center for Biotechnology Institute.

In an antibody molecule, three hypervariable regions of the light chain and three hypervariable regions of the heavy chain are arranged relative to each other in a three-dimensional space to form an antigen-binding surface. The antigen-binding surface is complementary to the three-dimensional surface of the bound antigen, and the three hypervariable regions of each heavy chain and light chain are referred as "complementarity determining region" or "CDR". The assignment of amino acids to each domain is defined by Kabat "Sequences of Proteins of Immunological Interest" (National Institutes of Health, Bethesda, Maryland (1987 and 1991)) or Chothia and Lesk (J. Mol. Biol. 196:901-917 (1987), Chothia et al., Nature 342:878-883 (1989)).

The "antibody" of the present disclosure refers to a polypeptide or a polypeptide complex that specifically recognizes and binds to an antigen. The antibody may be an intact antibody and any antigen-binding fragments or single chains thereof. The "antibody" of the present disclosure includes any protein or peptide containing at least a portion of an Ig molecule that has the biological activity of binding to an antigen. Examples of the "antibody" of the present disclosure include, but are not limited to, heavy or light chain CDRs or ligand binding portions thereof, heavy or light chain variable regions, heavy or light chain constant regions, framework regions, or any portion thereof.

The "antigen-binding fragment" of the present disclosure includes the following fragment with antigen-binding activity: Fab fragment, Fab' fragment, F(ab')₂ fragment, and Fv fragment and scFv fragment that bind to human CD47 or PD-L1. The Fv fragment comprises a first heavy chain variable region and a first light chain variable region of the antibody but no constant region, and it is the smallest antibody fragment with all antigen binding sites. Generally, Fv antibody also comprises a polypeptide linker between the VH and VL domains, and is able to form the structure required for antigen binding. Different linkers may also be utilized to link the two variable regions of antibody to form a polypeptide chain, which is called single-chain antibody or single-chain Fv (scFv). The anti-CD47 or anti-PD-L1 antibody of the present disclosure may be a single-chain variable fragment (scFv), which is derived from the single-chain polypeptide of an antibody and retains the ability to bind to antigen. Examples of scFv include antibody polypeptides generated by recombinant DNA techniques in which the Fv regions of immunoglobulin heavy chain (H chain) and light chain (L chain) fragments are linked via a spacer sequence. Various methods for preparing scFv are well known to those skilled in the art.

The antibody of the present disclosure refers to an immunoglobulin molecule or an immunologically active part thereof, that is, a molecule that contains antigen-binding sites that specifically bind to the antigen (through immunological reaction). "Specific binding" means that an antibody reacts with one or more antigenic determinants of an antigen but does not react with other polypeptides, or it binds to other polypeptides with very low affinity (Kd >10⁻⁶). Antibodies include but are not limited to polyclonal, monoclonal, chimeric, dAb (domain antibody), single chain, Fab, Fab' and F(ab')₂ fragment, Fv, scFv and Fab expression library. A monoclonal antibody (mAb) is the antibody obtained from a single cloned cell line, and the said cell line is not limited to eukaryotic, prokaryotic or phage cloned cell lines. A monoclonal antibody or antigen-binding fragment thereof can be obtained by recombination using, for example, hybridoma technology, recombination technology, phage display technology, and synthesis technology such as CDR grafting, or other existing technology.

The "murine-derived antibody" of the present disclosure is a monoclonal antibody against human CD47 produced according to the knowledge and skills in the art. During the production, the test subject is injected with the CD47 antigen, and then the hybridomas expressing the antibody with the desired sequence or functional property are isolated.

The "chimeric antibody" of the present disclosure is an antibody formed by fusing the variable regions of a murine-derived antibody with the constant regions of a human antibody, which can reduce the immune response induced by the murine-derived antibody. To establish a chimeric antibody, it is necessary to establish a hybridoma secreting murine-derived specific monoclonal antibodies first, clone the variable region genes from the mouse hybridoma cells, and then clone the constant region genes of the human antibody as needed, and chimeric genes formed by linking the mouse variable region with the human constant region genes are inserted into a human vector. Finally, the chimeric antibody is expressed in a eukaryotic system or a prokaryotic system.

The "humanized antibody" of the present disclosure is also called a CDR grafted antibody, which is the antibody produced by grafting mouse CDR sequences into the framework (FR) of a human antibody. Such framework sequences of variable region may be obtained from public DNA databases or public references, for example, from the ImMunoGeneTics (IMGT) website http://imgt.cines.fr or from the Journal of Immunoglobulin, 2001ISBN012441351.

The "bispecific antibody" as used herein refers to a monoclonal antibody that has binding sites for at least two different antigens.

The "peptide linker" as used herein may be those comprising any amino acid from 1 to 10, especially 2 to 50, and may comprise any kind of amino acids without any limitation.

### BRIEF DESCRIPTION OF DRAWINGS

FIG. 1 shows the results of the binding activity assay (ELISA) of the humanized anti-CD47 antibodies to monkey CD47.
FIG. 2 shows the results of the binding activity assay (ELISA) of the humanized anti-CD47 antibodies to human CD47.
FIG. 3 shows the results of the binding activity assay (ELISA) of the humanized anti-CD47 antibodies to CD47 on cell surface.
FIG. 4 shows the results of the hemagglutination test, wherein RBC represents the positive control and PBS represents the blank control.
FIG. 5 shows the results of the blocking activity of the humanized anti-CD47 antibodies detected by FACS.
FIG. 6 shows the results of the anti-tumor test of the humanized anti-CD47 antibody Hu34-39-PE in human gastric cancer NUGC-4 xenograft model.
FIG. 7 shows the results of the anti-tumor test of the humanized anti-CD47 antibody Hu26T-31-PE in human gastric cancer NUGC-4 xenograft model.
FIG. 8 shows a schematic diagram of the structure of bispecific antibody ScFab (HuPL7-21Ks/Hu34-39Hs).
FIG. 9 shows a schematic diagram of the sequence of ScFabHuPL7-21Ks.
FIG. 10 shows a schematic diagram of the sequence of ScFabHu34-39Hs.
FIG. 11 shows the results of the bispecific antibodies ScFab (HuPL7-21Ks/Hu34-39Hs) and ScFab (HuPL16-42Ks/Hu34-39Hs) binding to PD-L1 (A), competing with PD-1 (B), binding to CD47 (C), competing with SIRPα (D) and competing with CD80 (E).
FIG. 12 shows the results of the bispecific antibodies ScFab (HuPL7-21Ks/Hu34-39Hs) and ScFab (HuPL16-42Ks/Hu34-39Hs) binding to PD-L1 on cell surface (A), binding to CD47 on cell surface (B), blocking the binding of PD-1 to PD-L1 (C) and blocking the binding of SIRPα to CD47 (D) at the cellular level.
FIG. 13 shows the results of the bispecific antibodies ScFab (HuPL7-21Ks/Hu34-39Hs) and ScFab (HuPL16-42Ks/Hu34-39Hs) binding to both CD47 and PD-L1 on Raji-hPD-L1 cells (A, B) and blocking both CD47 and PD-L1 (B).
FIG. 14 shows the results of the bispecific antibody ScFab (HuPL7-21Ks/Hu34-39Hs) inhibiting the growth of transplanted tumors in mice.

### DETAILED DESCRIPTION

The following representative examples are used to illustrate the present disclosure better. The experimental methods without conditions indicated in the following examples are usually carried out according to conventional conditions, such as the antibody technology experiment manual and molecular cloning manual of Cold Spring Harbor, or according to the conditions recommended by the raw material or commodity manufacturers. The materials and reagents used in the examples are all commercially available unless otherwise specified.

### Example 1 Preparation of CD47 antigen protein and anti-CD47 positive control antibody

### 1. Construction of expression vector of antigen and positive control antibody

### (1) Construction of expression vector of antigen

A gene fragment encoding the full length of CD47 protein was synthesized, and the amino acid sequence is shown in SEQ ID NO: 41. This fragment was cloned into the eukaryotic expression plasmid pTargeT to generate the CD47 expression plasmid pTargeT-CD47.

The amino acid sequence of the extracellular region of the human CD47 protein was fused with the amino acid sequence of hIgG1-Fc or his-tag, and the designed amino acid sequences are shown in SEQ ID NO: 42 and SEQ ID NO: 43, respectively. After codon optimization of the above sequences encoding amino acids, the tagged extracellular region of CD47 protein coding fragments, CD47-hFc and CD47-his, were synthesized and cloned into the eukaryotic expression plasmid pHR respectively to generate expression plasmids pHR-CD47-hFc and pHR-CD47-his.

The amino acid sequence of the extracellular region of the human CD47 protein was fused with the amino acid sequence of mIgG1-Fc, and the designed amino acid sequence is shown in SEQ ID NO: 44. After codon optimization of the sequence, a complete expression plasmid pcDNA3.1(+)-TPA-CD47-mIgG1-Fc was constructed.

The sequence of SIRPα is shown in SEQ ID NO: 45. After codon optimization of the sequence, a complete expression plasmid pcDNA3.1(+)-SIRPα-myc-His was constructed.

### (2) Construction of expression vector of positive control antibody

The antibody AB6.12-IgG4P (abbreviated as AB06.12-4P hereinafter) disclosed in the patent application WO2013/119714 was used as a positive control antibody. The amino acid sequences of AB06.12-4P are as follows:
heavy chain amino acid sequence of AB06.12-4P is shown in SEQ ID NO: 46; and
light chain amino acid sequence of AB06.12-4P is shown in SEQ ID NO: 47.

The amino acid sequence corresponding to the above antibody sequences were artificially optimized to generate the heavy chain and light chain expression plasmids pcDNA3.1(+)-SHC025-hG4 and pcDNA3.1(+)-SHC025-hk of the positive control antibody AB06.12-4P. The heavy chain fragment was cloned into the eukaryotic expression plasmid pHR containing the light chain constant region of IgG4 to obtain the heavy chain eukaryotic expression plasmid pHR-SHC025-hG4-4PE and the light chain expression plasmid pcDNA3.1(+)-SHC025-hk of AB06.12-4P.

### 2. Expression and purification of antigen protein and positive control antibody

### (1) Construction of stable transgenic cell line of antigen protein

CHO-K1 cells (Shanghai Institute of Biochemistry and Cell Biology, Chinese Academy of Sciences) were electrotransfected with eukaryotic expression plasmid pTargeT-CD47 under a square pulse of 15msec at a voltage of 160V and then cultured in an incubator at 37°C with 5% CO₂. After 24h, the cells were subjected to selection with culture medium containing 500 µg/ml G418. After 16 days, the positive rate of cell pool was detected by FACS. The cells transfected with plasmid were plated (1×10⁶ cells/ml of cell density, 100 µl/well), and incubated with PE mouse anti-human CD47 antibody (BD, 556046). Flow cytometer (BD, FACSJazz) was used to read the mean value at a wavelength of 585nm, and data analysis was performed using GraphPad. The positive cell lines were subcloned, and a CHO-K1 cell line was selected, which expressed CD47 at a high level and was named CHO-K1-E5.

### (2) Expression of tagged antigen protein and positive control antibody

293F cells were inoculated into a 1L cell culture flask with a density of 0.5×10⁶ cells/ml. Fresh and pre-warmed FreeStyle 293 expression medium was added to make the total volume 250mL. The cells were cultured in a humidified CO₂ incubator at 37°C with 8% CO₂ overnight. 500µl of 1 mg/ml PEI solution was added to 8.5 mL FreeStyle 293 expression medium and mixed well. 250µg of plasmid to be transfected was added to 8.5 ml FreeStyle 293 expression medium and mixed well, wherein, the plasmids encoding tagged antigen, pHR-CD47-hFc, pHR-CD47-his, pcDNA3.1(+)-TPA-CD47-mIgG1-Fc and pcDNA3.1(+)-SIRPα-myc-His, were transfected respectively. The heavy chain plasmid pHR-SHC025-hG4-4PE and the light chain plasmid pcDNA3.1(+)-SHC025-hk of positive control antibody AB06.12-4P were co-transfected. The FreeStyle 293 expression medium containing PEI was added to the expression medium containing plasmids and mixed well, then the mixture was added to the cells, and the cells were cultured in a humidified CO₂ incubator at 37°C with 8% CO₂. The cells were fed on the 1st and 3rd day after cell transfection with 2.5 ml of 200 mM glutamine and 5 ml of 180 g/L glucose per flask. The cell culture supernatant was collected when the cell viability dropped to 65%~75%. The cell culture was centrifuged at 1,500 rpm for 5 min to collect the supernatant. The supernatants were centrifuged at 8,000 rpm for 20 min to collect the supernatant again.

### (3) Affinity chromatography purification

Different affinity chromatography columns were used to perform purification by using AKTA machine (GE, AKTA pure-150) according to the properties of the proteins (see Table 1 for affinity chromatography columns suitable for different proteins). The specific purification steps are as follows.

**Table 1 Affinity chromatography columns suitable for different proteins**

| Protein | Column | Brand | Model |
|---|---|---|---|
| Murine monoclonal antibody (hybridoma)/CD47-mFc | Protein G prepacked column | Bestchrom | Ezfast Protein G 4FF |
| CD47-his/ SIRPα-myc-His | NI prepacked column | GE | His Trap, HP 5ml |
| CD47-hFc/chimeric antibody, humanized antibody | Protein A prepacked column | GE | Hi Trap, Mabselect SuRe 5 ml |
| | Protein A self-packed column | GE | Mabselect LX 18 ml |

### Cleaning

The equipment and pipelines were cleaned with ultrapure water for 2min with a flow rate of 10mL/min, and then the chromatography system was cleaned with 0.1M NaOH.

### Column connection

The chromatography column was connected to the chromatography equipment and rinsed with ultrapure water for 5min, and then the chromatography system was rinsed with 0.1M NaOH for 30min with a retention time of 5min.

### Equilibration

Five CVs (column volume) of 20mM PB + 0.15M NaCl, pH 7.2 was used to equilibrate the column.

### Sample loading

The supernatant from cell culture was loaded to the column with a retention time of 5 min.

### Post-equilibration

Five CVs 20mM PB+ 0.15M NaCl, pH 7.2 was used to equilibrate.

### Elution

Elution was performed with 50 mM acetic acid (pH=3.4), for a retention time of 5 min. Collection started when UV280 reached about 50 mAu, and stopped when UV280 dropped to about 50 mAu. The sample was adjusted to the pH of 7.0 with 1M Tris-HCl (pH 9.0).

### Re-equilibration

Three CVs of 20mM PB+ 0.15M NaCl, pH 7.2, was used to equilibrate with a retention time of 5min.

### On-column Cleaning

Cleaning was performed with 0.1M NaOH for 30 min with a retention time of 5 min.

### Cleaning and preservation

Cleaning was performed with purified water for 10 minutes, and then 2 CVs of 20% ethanol.

### Example 2 Preparation of anti-CD47 monoclonal antibodies

### 1. Preparation of hybridomas

### (1) Animal immunization

The experimental SJL mice were immunized with different tagged CD47 proteins together with adjuvants. 50µg of antigen was used for the first shoot, and 25µg of antigen was used for the subsequent immunization.

The immune adjuvants used in the experiments may be QuickAntibody-Mouse5W (Beijing Biodragon immunotech. Co., Ltd.), TiterMax (Sigma), CpG (GenScript Biotechnology Co., Ltd.), or Alum (thermo) adjuvant. Different tagged CD47 protein samples were added dropwise to the adjuvant solution with vortex to mix thoroughly. The dosages of the adjuvant were referred to the instructions. After the mixture was mixed well and formed a water-in-oil emulsion, the SJL mice were immunized.

Cell lines expressing high level of CD47, such as CCRF-CEM and CHO-K1-E5, were also used to immunize mice to produce antibodies. The cultured human acute lymphoblastic leukemia cells (CCRF-CEM) and CHO-K1-E5 (positive cell line obtained in Example 1) were treated with trypsin and then centrifuged at 1,000 rpm for 5 min. The supernatant was discarded and the cell pellets were resuspended in PBS. Part of the cell sample was taken out for cell counting, and the remaining cell sample was centrifuged at 1,000 rpm for 5 min. The supernatant was discarded and the cell pellet was resuspended in PBS. An appropriate amount of PBS was added to obtain a cell suspension of 1×10⁸ cells/ml. Each mouse in the experimental group was immunized with 1×10⁷ cells.

The immunization protocol is shown in Table 2.

**Table 2 Mouse Immunization Protocol**

| Group | Antigen | Adjuvant | Route^{∗} |
|---|---|---|---|
| 1 | PBS | None | |
| 2 | CD47-his/CD47-mFc | Quick Antibody-Mouse5W | i.m. |
| 3 | CD47-his/CD47-mFc | Titer Max/CpG/Alum | s.c./i.m. |
| 4 | CD47-mFc | Quick Antibody-Mouse5W | i.m. |
| 5 | CD47-mFc | Titer Max/CpG/Alum | s.c./i.m. |
| 6 | CCRF -CEM/CHO- K1-E5 | None | i.p. |

| | | | |
|---|---|---|---|
| ^{∗} i.m.: intramuscular injection; s.c.: subcutaneous injection; i.p.: intraperitoneal injection. | | | |

### (2) Hybridoma fusion

Acquisition and preparation of spleen cells. The mice after booster immunization were sacrificed and soaked in 75% alcohol. The spleen was dissected out, ground with a grinding rod, and filtered through a cell strainer to prepare a single cell suspension. The spleen cell suspension was centrifuged at 2,000 rpm for 5 min, and the supernatant was discarded. 2mL red blood cell lysate was added to lyse red blood cells at room temperature for 2 min and PBS was added to reach 20 mL. After centrifugation at 1,500 rpm for 7 min, the supernatant was discarded. Viable cells were counted after resuspension. The Sp2/0 cells in the culture flask were collected and after centrifuged at 1,000 rpm for 5 min, the supernatant was discarded. Viable cells were counted after resuspension. The spleen cells were mixed with Sp2/0 cells at a ratio of 1:1 and subjected to centrifugation at 1,500 rpm for 7 min, the supernatant was discarded. The cells were resuspended in 20 mL electroporation buffer. After centrifugation at 1,500 rpm for 7 min, the supernatant was discarded and the step was repeated once. The cells were resuspended with an appropriate amount of electroporation buffer to ensure the cell concentration of about 2×10⁷ cells/mL. The cell suspension was added to a 9mL electroporation tank for fusion. After fusion, the cell suspension was transferred to 15mL RPMI 1640 complete medium containing 20% FBS and then left at room temperature for 20 min. The fused cells were resuspended with RPMI 1640 medium containing 1×HAT, 1×BIOMYC3, and 20% FBS. The cell suspension was added to several 96-well cell culture plates at 100 µl/well to ensure that the cell volume per well was about 4×10⁴ cells/well, and the plates was placed in a 37°C cell incubator. After 5 days, additional 100 µL of RPMI 1640 complete medium containing 20% FBS, 1×HAT, and 1×BIOMYC-3 was added to each well.

### (3) Screening of hybridoma and subcloning

After one week of fusion, the supernatants of culture were collected and used for screening the hybridoma supernatants that can bind to CD47-his protein or CD47 on cell surface by ELISA. CD47-his was used to screen for antibodies against CD47 instead of hFc and mFc. The ability of the hybridoma supernatant to block the CD47-SIRPα interaction was analyzed by ELISA. SIRPα-myc-his was coated on ELISA plates. The mixture of recombinant humanized CD47-hFc and hybridoma supernatant was added and incubated for 2 h. HRP-labeled anti-human IgG Fc specific antibody (Jackson Immuno Research) was added and incubated for 1 h. Microplate reader was used to detect absorbance at 450nm. The hybridoma parent clones showing binding and blocking activities in the screening experiments were expanded. The binding and blocking activities were retested, and the hybridoma positive clones with binding and blocking activities were obtained by screening again.

The positive cell lines were subcloned by the limiting dilution method. After one week of culture, the binding activity to CD47 and the activity of blocking the CD47-SIRPα interaction of the supernatants were detected by ELISA. Three cell lines that showed positive results in the above two tests were obtained, respectively named as SHC025-26, SHC025-34 and SHC025-58.

### 2. Identification of antibody subtypes

The antibody subtypes were identified according to the instructions of the mouse antibody subtype identification kit "SBA Clonotyping Systerm-C57BL/6-HRP" (SouthernBiotech, Cat. No. 5300-05B). The results are shown in Table 3.

**Table 3 Identification results of antibody subtypes**

| Antibody | Antibody subtype |
|---|---|
| SHC025-26 | IgG1/k |
| SHC025-34 | IgG2c/k |
| SHC025-58 | IgG2b/k |

### 3. Preparation of monoclonal antibodies

According to the activity analysis results of the supernatants from cell culture, the parent clones of monoclonal antibodies SHC025-26, SHC025-34, and SHC025-58 were identified and expanded. The culture medium was 1640 medium containing 10% fetal bovine serum, 1x NAEE, 1x sodium pyruvate, and 1% penicillin-streptomycin double antibiotics. When the cell confluence was >80%, the cells were subcultured and expanded. 50 ml of the supernatant was collected and the antibody was purified. The obtained antibody was subjected to SDS-PAGE gel electrophoresis and showed a good purity.

### 4. Sequencing of monoclonal antibodies

The subcloned positive hybridomas were expanded, and an appropriate amount of cells was used for total RNA extraction according to the instructions of RNeasy Plus Mini Kit (Qiagen, 74134). The first strand of cDNA was synthesized using Prime Script 1st strand cDNA Synthesis Kit (Takara, 6110A).

Specific primers were designed according to the variable region of the mouse antibody subtype, and 5' end of the primers contained the homologous arm sequence for homologous recombination with the eukaryotic expression vector. PCR amplification for the variable region of antibodies was performed using cDNA as a template to obtain the gene fragments of the light chain variable region and heavy chain variable region of the mouse antibody respectively. The design of primers refers to references: 1. Anke Krebber, Susanne Bornhauser, Jorg Burmester etal. Reliable cloning of functional antibody variable domains from hybridomas and spleen cell repertoires employing a reengineered phage display system. Journal of Immunological Methods, 1997, 201: 35-55; 2. Simon KorenMiha KosmačAnja Colja Venturini etal. Antibody variable-region sequencing as a method for hybridoma cell-line authentication, 2008, 78: 1071-1078. DNA sequencing was performed and the results are shown in Table 4.

**Table 4 Sequence table of anti-CD47 murine-derived monoclonal antibody**

| Antibody | Amino acid sequence of the heavy chain variable region | Amino acid sequence of the light chain variable region |
|---|---|---|
| SHC025-26 | SEQ ID NO: 21 | SEQ ID NO: 24 |
| SHC025-34 | SEQ ID NO: 22 | SEQ ID NO: 25 |
| SHC025-58 | SEQ ID NO: 23 | SEQ ID NO: 26 |

For the antibody SHC025-34, the CDR sequences of VH: the sequences of CDR1, CDR2 and CDR3 are set forth in SEQ ID NOs: 4, 5 and 6, respectively; the CDR sequences of VL: the sequences of CDR1, CDR2 and CDR3 are set forth in SEQ ID NOs: 15, 16 and 17, respectively.

### Example 3 Construction of anti-CD47 chimeric antibodies

The purified gene fragments of the light chain and the heavy chain variable regions of the mouse antibody (see Example 1 for the purification steps) were respectively co-transformed into *Escherichia coli* DH5α competent cells with the linearized eukaryotic expression plasmid containing the light chain constant region or the heavy chain constant region of the human antibody. The mixture was spread evenly on the surface of the agar plates containing the corresponding antibiotics. The agar plates were incubated in a 37°C constant temperature incubator overnight, and then several single colonies were picked out for DNA sequencing. The chimeric antibodies with correct sequences were named SHC025-26CHI, SHC025-34CHI, and SHC025-58CHI.

The positive clones with correct sequences were inoculated in 2×YT liquid medium containing the corresponding antibiotics and cultured at 37°C for more than 12 hours with shaking. The bacterial cells were collected for plasmid extraction to obtain the plasmids expressing the light chain and the heavy chain of the chimeric antibody. The concentration and purity of the plasmids were detected by a nucleic acid quantitative analyzer.

The plasmids for chimeric antibodies were transfected into HEK293E cells, and the antibodies were expressed and purified. The purity, activity and affinity were tested and analyzed.

By sequencing, it was found that there was one cysteine in the heavy chain CDR position 118 of SHC025-26 and one cysteine in the light chain CDR position 56 of SHC025-58. During the antibody expression, the cysteine in CDR region would randomly pair with the other cysteine on the antibody molecule to form a disulfide bridge, which thereby would greatly affect the purity of the antibody. To solve this problem, the amino acid sequences of SHC025-26CHI and SHC025-58CHI were modified as follows: C118 of SHC025-26CHI heavy chain was mutated to T and the obtained antibody was named SHC025-26CHI-T; C56 of SHC025-58CHI light chain was mutated to A and the obtained antibody was named SHC025-58CHI-A. The mutant sequences were constructed by site-directed mutagenesis. The results of the chimeric antibody sequencing are shown in Table 5.

**Table 5 Sequences of anti-CD47 chimeric antibodies**

| Chimeric antibody | Amino acid sequence of VH | Amino acid sequence of VL |
|---|---|---|
| SHC025-26CHI | SEQ ID NO: 21 | SEQ ID NO: 24 |
| SHC025-26CHI-T | SEQ ID NO: 27 | SEQ ID NO: 24 |
| SHC025-34CHI | SEQ ID NO: 22 | SEQ ID NO: 25 |
| SHC025-58CHI | SEQ ID NO: 23 | SEQ ID NO: 26 |
| SHC025-58CHI-A | SEQ ID NO: 23 | SEQ ID NO: 28 |

For the antibody SHC025-34CHI: the sequences of CDR1, CDR2 and CDR3 of VH are set forth in SEQ ID NOs: 4, 5 and 6, respectively; the sequences of CDR1, CDR2 and CDR3 of VL are set forth in SEQ ID NOs: 15, 16 and 17, respectively.

### Example 4 Construction and production of humanized anti-CD47 antibodies

Based on the results of activity analysis and affinity *KD* value of chimeric antibodies, SHC025-34CHI, SHC025-58CHI-A, and SHC025-26CHI-T were modified to humanized antibodies.

To construct the humanized antibodies, the variable regions of SHC025-34CHI, SHC025-58CHI-A, and SHC025-26CHI-T antibody were compared with the mouse antibody sequences in the ImMunoGeneTics (IMGT) to determine their murine-derived germlines. After homology comparison, it was found that the FR region sequences of the heavy chain variable region of SHC025-34CHI, SHC025-58CHI-A, and SHC025-26CHI-T antibody were the most similar to the human antibody germline genes IGHV1-8^{∗}01, IGHV3-21^{∗}04, and IGHV1-2^{∗}02 respectively; the FR sequences of the light chain variable region of the antibodies were the most similar to the human antibody germline genes IGKV3-11^{∗}01, IGKV1-5^{∗}01 and IGKV4-1^{∗}01 respectively. With the framework region sequence FR1-FR3 of SHC025-34CHI/SHC025-58CHI-A antibody as templates, full human framework regions with similar 3D structure but low immunogenicity were screened in the human framework region library to replace FR1-FR3 sequence of SHC025-34CHI/SHC025-58CHI-A. The full-length sequences of the heavy/light chain were 3D modeled and compared structurally with the heavy/light chain sequences of the original antibodies. Considering the antigenicity and 3D structural similarity, 6 humanized heavy chain variable regions (see SEQ ID NOs: 48, 49, 50, 51, 52, and 53) and 4 humanized light chain variable regions (see SEQ ID NOs: 54, 55, 56, and 57) of SHC025-34CHI, and 6 humanized heavy chain variable regions (see SEQ ID NOs: 58, 59, 60, 61, 62, and 63) and 5 humanized light chain variable regions (see SEQ ID NOs: 64, 65, 66, 67, and 68) of SHC025-58CHI-A were ultimately selected for further optimization. More than 95% of non-CDR regions of SHC025-34CHI or SHC025-58CHI-A antibody were humanized. The variable region sequences of the heavy and light chain of SHC025-26CHI-T were used to perform structural alignment analysis in Protein Data Bank. The FR1-FR3 sequences with the highest similarity were selected to replace the murine-derived sequences, and the amino acid sites that displayed key role in structure stabilization of the antibody in the structural simulation were mutated back to murine-derived amino acid residues. Finally, 4 humanized heavy chain variable regions (see SEQ ID NOs: 69, 70, 71, and 72) and 2 humanized light chain variable regions (see SEQ ID NOs: 73 and 74) of SHC025-26CHI-T were obtained.

The amino acid sequences of the light chain and heavy chain variable region of humanized antibody obtained above were reversely transcribed into their corresponding nucleotide sequences, and oligonucleotide fragments containing complementary sequences between adjacent fragments were generated. The oligonucleotide fragments were annealed and assembled by Overlap PCR. Then nucleotide fragments of the entire light chain and heavy chain variable regions were amplified using specific primers (5' end contained the homologous arm sequence for homologous recombination with the eukaryotic expression vector). The purified nucleotide fragments of the light chain variable region were co-transformed into *Escherichia coli* DH5α competent cells with the linearized eukaryotic expression plasmid containing light chain constant region of IgG4. The purified nucleotide fragments of the heavy chain variable region were co-transformed into *Escherichia coli* DH5α competent cells with the eukaryotic expression plasmid containing heavy chain constant region of IgG4 containing S228P/L235E mutation. The competent cells with the transformed plasmid were spread evenly on the surface of the agar plates containing the corresponding antibiotics. The agar plates were incubated in a 37°C constant temperature incubator overnight, and then several single colonies were picked out for DNA sequencing.

The positive clones with correct sequences were inoculated in 2×YT liquid medium containing the corresponding antibiotics and cultured at 37°C with shaking for more than 12 hours. The bacterial cells were collected for plasmid extraction to obtain the expression plasmids for the light chain and the heavy chain of humanized antibodies. The concentration and purity of the plasmids were detected by a nucleic acid quantitative analyzer.

The plasmids were transfected into HEK293E cells, and a large number of antibodies were expressed and purified. The purity, activity and affinity were tested and analyzed.

The humanized antibodies with good purity, activity and affinity were selected, and named as Hu26T-31-PE, Hu34-39-PE, and Hu58A-14-PE. The sequences are shown in Table 6. The sources of humanized antibodies are shown in Table 7.

**Table 6 Sequence table of humanized anti-CD47 antibodies**

| Humanized antibody | Amino acid sequence | | Nucleotide sequence | |
|---|---|---|---|---|
| | VH | VL | VH | VL |
| Hu26T-31-PE | SEQ ID NO: 29 | SEQ ID NO: 32 | SEQ ID NO: 35 | SEQ ID NO: 38 |
| Hu34-39-PE | SEQ ID NO: 30 | SEQ ID NO: 33 | SEQ ID NO: 36 | SEQ ID NO: 39 |
| Hu58A-14-PE | SEQ ID NO: 31 | SEQ ID NO: 34 | SEQ ID NO: 37 | SEQ ID NO: 40 |

For the antibody Hu34-39-PE, the sequences of CDR1, CDR2 and CDR3 of VH are set forth in SEQ ID NOs: 4, 5 and 6, respectively; the sequences of CDR1, CDR2 and CDR3 of VL are set forth in SEQ IDNOs: 15, 16 and 17, respectively.

**Table 7 Information of humanized sequences**

| Heavy chain | |
|---|---|
| Source of humanized sequence | Recombinant humanized sequence |
| X62106 Homsap IGHV1-2^{∗}02 F | SEQ ID NO: 29 |
| X92343 Homsap IGHV1-46^{∗}01 F | SEQ ID NO: 30 |
| HM855688 Homsap IGHV3-21^{∗}04 F | SEQ ID NO: 31 |

| Light chain | |
|---|---|
| Source of humanized sequence | Recombinant humanized sequence |
| X97473 Homsap IGLV3-9^{∗}01 F | SEQ ID NO: 32 |
| X71966 Homsap IGLV3-21^{∗}01 F | SEQ ID NO: 33 |
| Z73648 Homsap IGLV4-69^{∗}01 F | SEQ ID NO: 34 |

### Example 5 Binding activity assay of anti-CD47 antibodies to monkey CD47 (by ELISA)

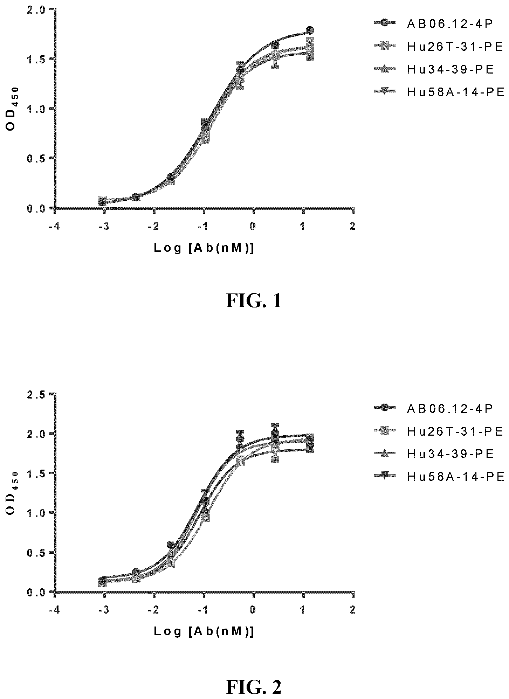

The binding activity of antibodies was analyzed by protein based ELISA. Cynomolgus CD47-His (0.1 µg/well, ACRO Biosystems, Cat. No. CD7-C52H1-50µg) was coated on 96-well ELISA plates. The anti-CD47 antibodies of the present disclosure was used as the primary antibody and added to the ELISA plates in 5-fold gradient dilution with a total of 8 concentrations: 2000 ng/mL, 400 ng/mL, 80 ng/mL, 16 ng/mL, 3.2 ng/mL, 0.64 ng/mL, 0.128 ng/mL, and 0 ng/mL respectively. The plates were incubated at 37°C for 1.5h. AB06.12-4P was used as the positive control antibody and Anti-Human IgG HRP (Jackson, 109-035-003, 1:10000) was used as the secondary antibody. Color developing solution TMB (3,3',5,5'-tetramethylbenzidine) was added to the plate, and microplate reader (Thermo, Multiskan FC) was used to read OD450 value after termination of the reaction. EC₅₀ was generated by GraphPad. The result is shown in FIG. 1.

The experimental results show that humanized anti-CD47 antibodies Hu26T-31-PE, Hu34-39-PE and Hu58A-14-PE of the present disclosure can bind to cynomolgus CD47, and the binding ability is equivalent to that of the positive control antibody AB06.12-4P.

### Example 6 Binding activity assay of anti-CD47 antibodies to human CD47 (by ELISA)

The binding activity of antibodies was analyzed by ELISA. Human CD47-His protein (0.1 µg/well, prepared in Examples 1 and 2) was coated on 96-well ELISA plates, and the ELISA plates were incubated at 37°C for 2h. After washing 3 times with 1×PBST, the ELISA plates were blocked with 5% non-fat milk at 4°C overnight. The plates were washed 3 times with 1×PBST. The anti-CD47 antibodies of the present disclosure were used as the primary antibody and added to the ELISA plates at 5-fold gradient dilution with a total of 8 concentrations: 2000 ng/mL, 400 ng/mL, 80 ng/mL, 16 ng/mL, 3.2 ng/mL, 0.64 ng/mL, 0.128 ng/mL, and 0 ng/mL respectively. The plates were incubated at 37°C for 1.5h. The plates were washed 3 times with 1×PBST. AB06.12-4P was used as the positive control antibody and Anti-Human IgG HRP (Jackson, 109-035-003, 1:10000) was used as the secondary antibody. Then the plates were incubated at 37°C for 40 min. Color developing solution TMB was added after the plates were washed 5 times with 1×PBST, and microplate reader (Thermo, Multiskan FC) was used to read OD450 value after termination of the reaction. EC₅₀ was generated by GraphPad. The result is shown in FIG. 2.

The experimental results show that humanized anti-CD47 antibodies Hu26T-31-PE, Hu34-39-PE and Hu58A-14-PE of the present disclosure can bind to human CD47, and the binding ability is equivalent to that of the positive control antibody AB06.12-4P.

### Example 7 Binding activity assay of anti-CD47 antibodies to CD47 on cell surface (by ELISA)

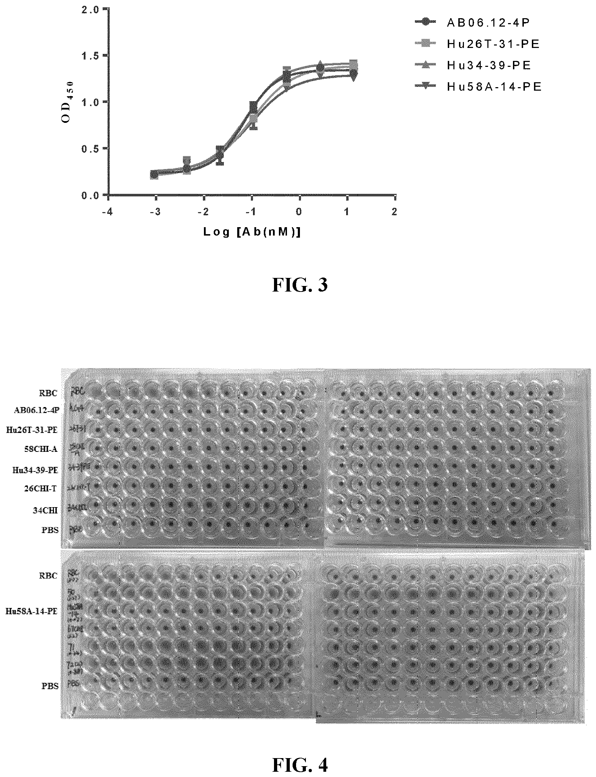

The binding activity of antibodies was analyzed by cell based ELISA. CHO-K1-E5 cells were plated at 1×10⁵ cells per well and cultured overnight at 37°C and 5% CO₂. On the second day, the cells were fixed with 4% paraformaldehyde, then blocked with non-fat milk for 1 h, and later washed gently with 1×PBS. The anti-CD47 antibody of the present disclosure was used as the primary antibody and added to the plates in 5-fold gradient dilution with a total of 8 concentrations: 2000 ng/mL, 400 ng/mL, 80 ng/mL, 16 ng/mL, 3.2 ng/mL, 0.64 ng/mL, 0.128 ng/mL, and 0 ng/mL respectively. The plates were incubated at 37°C for 1.5h. AB06.12-4P was used as the positive control antibody and Anti-Human IgG HRP (Jackson, 109-035-003, 1:10,000) was used as the secondary antibody. Color developing solution TMB was added, and microplate reader (Thermo, Multiskan FC) was used to read OD450 value after termination. EC₅₀ was generated by GraphPad. The result is shown in FIG. 3.

The experimental results show that humanized anti-CD47 antibodies Hu26T-31-PE, Hu34-39-PE and Hu58A-14-PE of the present disclosure can bind to CD47 on cell surface, and the binding ability is equivalent to that of the positive control antibody AB06.12-4P.

### Example 8 Affinity assay of anti-CD47 antibodies to human CD47 protein

The affinity of the humanized anti-CD47 antibodies prepared in Examples 1 and 2 to the antigen CD47(19-136)-hFC was determined by Fortebio Octet. First, the antigen CD47(19-136)-hFc was labeled with biotin, and then put into 10kD cut-off ultrafiltration tube with PBS for desalting by centrifugation. This step was repeated 3-4 times. The actual concentration of the biotin-labeled antigen (CD47-hFc-Biotin) was determined by Nanodrop machine. CD47-hFc-Biotin was diluted with SD buffer (0.02% Tween20 + 0.1% BSA solution) to a concentration of 5µg/ml. The humanized anti-CD47 antibody was diluted with SD buffer in 4-fold gradient dilution to make the concentrations of 10µg /ml, 2.5µg/ml, 0.625µg/ml and 0µg/ml. SA sensor was used to solidify the antigen, and affinity assay was performed according to the manual of fortebio Octet RED96. The specific parameters and experimental results are shown in Table 8.

**Table 8 Affinity assay of antibodies to human CD47 protein**

| Antibody | KD (M) | kon (1/Ms) | kdis (1/s) |
|---|---|---|---|
| Hu26T-31-PE | 5.87E-11^{∗∗} | 1.04E+06 | 6.13E-05 |
| Hu34-39-PE | 1.13E-10 | 6.69E+06 | 7.58E-04 |
| Hu58A-14-PE | 9.46E-11 | 1.78E+06 | 1.68E-04 |
| AB06.12-4P | 1.01E-10 | 4.80E+06 | 4.85E-04 |

The experimental results show that the humanized anti-CD47antibody Hu26T-31-PE has significantly higher affinity for binding to human CD47 protein than the positive control antibody.

### Example 9 Hemagglutination test of anti-CD47 antibodies

5 mL blood sample was added to 40 mL PBS. The mixture was centrifuged at 2000 rpm for 5 min and the supernatant was discarded. The cell pellet was washed three times with PBS and then resuspended in PBS. According to the hematocrit, a 2% red blood cell suspension was prepared. The initial concentration of the antibody to be analyzed was 1-20 µM at a 2-fold dilution, a total of 24 concentration gradients. In round-bottom 96-well plates, 50 µL of the above-mentioned antibodies of different concentrations was added, and then 50ul of the 2% red blood cell suspension was added. The mixture was mixed well, placed at room temperature and monitored for agglutination after 2h. Rabbit polyclonal RBC antibody (Rockland, 109-4139) was used as a positive control for hemagglutination and the results are shown in FIG. 4. As shown in FIG. 4, the concentration of the antibodies (rabbit polyclonal RBC antibody, AB06.12-4P antibody and the test antibodies of the present disclosure) from left to right in the 96-well plates were diluted in a 2-fold gradient starting from 20uM. RBC indicated the positive control group, in which rabbit polyclonal RBC antibody caused significant hemagglutination), and PBS indicated the blank control group. If there is no cell agglutination, the red blood cells will fall to the bottom of the well as a small dot with smooth edge. Dot with slightly vague edge indicates agglutination of a small amount of red blood cells. If the red blood cells form flaky shape and cover the whole bottom of the well, this indicates agglutination of most of the red blood cells.

It has been reported that the anti-CD47 antibody Hu5F9-G4 disclosed in the patent application WO2011/143624 can cause significant agglutination of red blood cells within the same concentration range, which is a common undesirable property of anti-CD47 antibodies. However, under the same conditions, Hu26T-31-PE, Hu34-39-PE, and Hu58A-14-PE of the present disclosure did not cause hemagglutination as shown in the experimental results, indicating that the antibodies of the present disclosure are significantly superior to the Hu5F9-G4 antibody in this respect.

### Example 10 Blocking activity assay of anti-CD47 antibodies against CD47

The ability of the anti-CD47 antibody provided by the present disclosure to block SIRPa from binding to CD47 on cell surface was detected by FACS.

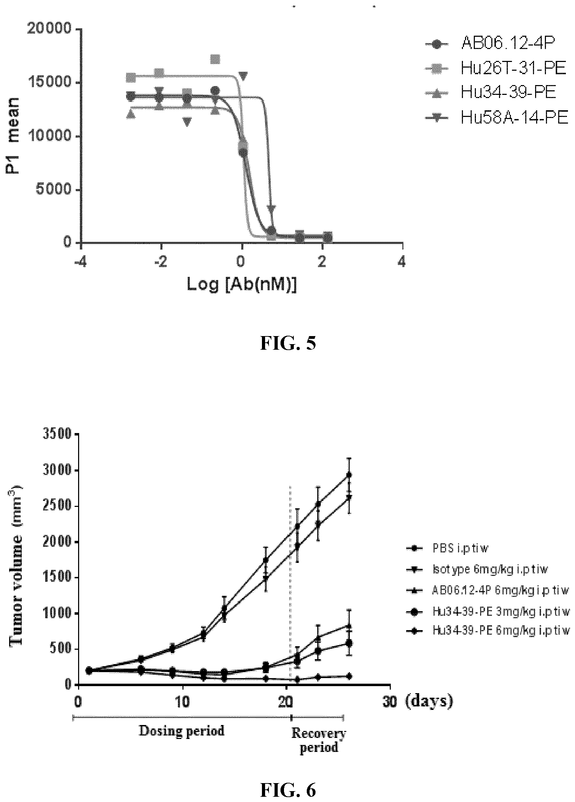

CHO-K1-E5 cells positive for CD47 were used as a CD47 provider. In the presence of a serially diluted anti-CD47 antibody, the binding of CD47 to SIRPa was monitored. PE Streptavidin (Biolegend, 405203, 1:200) was used as the secondary antibody to monitor the changes of SIRPa-Biotin, and AB06.12-4P was used as a positive control which blocked SIRPa from binding to CD47 on cell surface. Flow cytometer (BD, FACSJazz) was used to read the mean value at a wavelength of 585nm and IC₅₀ was generated by GraphPad. The result is shown in FIG. 5.

The experimental results show that the blocking activity ranking from high to low is: Hu26T-31-PE≥Hu34-39-PE≥AB06.12-P>Hu58A-14-PE.

### Example 11 Anti-tumor test of anti-CD47 antibodies in human gastric cancer NUGC-4 xenograft model

### 1. Experimental materials

### (1) Experimental cells and animals

NUGC-4 human gastric cancer cells were purchased from American Type Culture Collection (ATCC).

NOD-Scid mice, female, 5-8 weeks old, weighing 18-20 grams, were purchased from Shanghai Lingchang Biotechnology Co., Ltd.

### (2) Test samples and controls

The reference antibody isotype IgG4 (Cat. No. AB170091) was purchased from Crown Bioscience Co., Ltd. and used as a negative control.

Before the test, the humanized anti-CD47 antibody of the present disclosure was prepared at two concentrations of 0.6 mg/mL and 0.3 mg/mL in PBS, and Isotype IgG4 and AB 06.12-4P were prepared at 0.6 mg/mL.

### (3) Experimental methods

NUGC-4 human gastric cancer cells were cultured with RMPI1640 medium containing 10% fetal bovine serum, 100 U/mL penicillin and 100 µg/mL streptomycin in a 37°C, 5% CO₂ incubator. The cells were digested, treated with 2 mL 1×EDTA solution and passaged once a week. When the cell confluence reached 80%-90%, the cells were collected, counted, and seeded. PBS containing 5×10⁶ cells was mixed with 100 uL Matrigel (final volume of 200 µL). The mouse was injected with the mixture on the right back side, 5×10⁶ cells /mouse. The mice were grouped when the tumor grew to a volume of 150-200 mm³ and administered intraperitoneally with the test sample three times per week. The tumor diameter was measured with a vernier caliper three times per week and the tumor volume was calculated by a calculation equation of V= 0.5a×b², where a and b represent the long and short diameters of the tumor, respectively. The anti-tumor efficacy of antibodies was evaluated by the relative tumor growth rate T/C (%). The calculation equation of the relative tumor growth rate T/C (%) is as follows: T/C% = TRTV/CRTV × 100% (TRTV: treatment group RTV; CRTV: negative control group RTV). RTV = V21/V0, where V0 is the tumor volume measured at the time of being grouped and administered (Day 0), and V21 is the tumor volume measured at the 21th day of administration (Day 21). The tumor volumes on the last day (Day 21) of the administration group and the vehicle group were analyzed by t-test using GraphPad Prism. The results are shown in Table 9.

**Table 9 Anti-tumor test results in human gastric cancer NUGC-4 xenograft model**

| Antibody | Dose (mg/kg) | T/C (%) | p (vs. Isotype IgG4) |
|---|---|---|---|
| Isotype IgG4 | 6 | 86.61 | - |
| AB06.12-4P | 6 | 18.82 | ∗∗ |
| Hu26T-31-PE | 3 | 12.24 | ∗∗∗ |
| Hu26T-31-PE | 6 | 2.96 | ∗∗∗ |
| Hu34-39-PE | 3 | 15.29 | ∗∗∗ |
| Hu34-39-PE | 6 | 3.45 | ∗∗∗ |

| | | | |
|---|---|---|---|
| Comparing with Isotype IgG4, ^{∗∗∗} indicates p<0.001; ^{∗∗} indicates p<0.005. | | | |

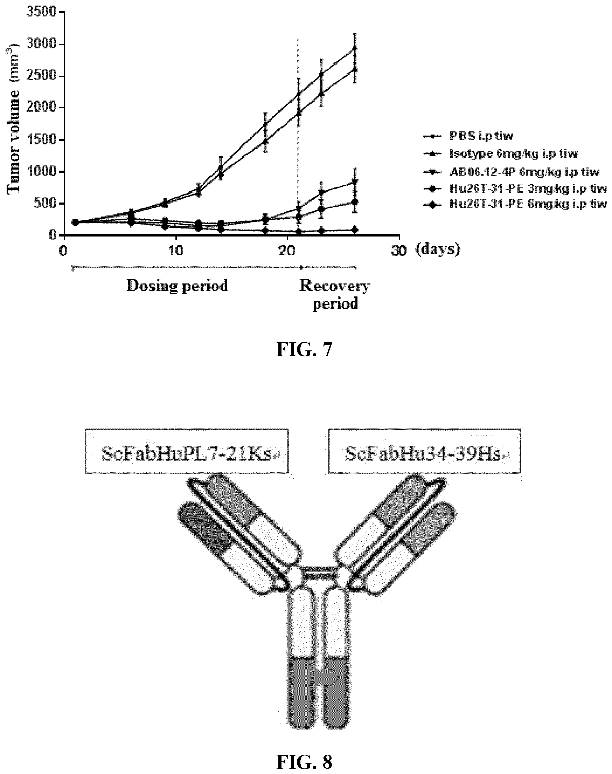

The experimental results show that the antibodies of the present disclosure have a significant anti-tumor effect in NOD-SCID mice xenograft model inoculated with human gastric cancer NUGC-4 cells. Hu26T-31-PE and Hu34-39-PE at a dose of 3 mg/kg show equivalent tumor-suppressive effect as the reference antibody AB06.12-4P at a dose of 6 mg/kg, while Hu26T-31-PE and Hu34-39-PE at a dose of 6 mg/kg show a better tumor-suppressive effect than the reference antibody AB06.12-4P at a dose of 6mg/kg. One week after drug withdrawal, tumor recurrence occurred in the group of reference antibody at a dose of 6mg/kg, while no recurrence occurred in the group of Hu26T-31-PE or Hu34-39-PE (FIG. 6 and FIG. 7). It indicates that the anti-CD47 antibodies of the present disclosure have unexpectedly better effect on inhibiting tumor growth.

### Example 12 Production of anti-human PD-L1 antibodies

### 1. Animal immunization

Experimental BALB/c and SJL mice and SD rats were immunized with mFc-tagged PD-L1 antigen protein (purchased from ACROBiosystems, Beijing) together with adjuvants.

The immune adjuvant was Freund's Adjuvant, Complete (SIGMA, F5881-10ML) for the first time, and Freund's Adjuvant, Incomplete (SIGMA, F5506-10ML) for the later stages. Different tagged PD-L1 protein samples were added dropwise to the adjuvant solution with vortex to mix thoroughly. The dosages of the adjuvant were referred to the instructions. After the mixture was mixed well to form a water-in-oil emulsion, mice or rats were immunized. The immunization protocol is shown in Table 10.

**Table 10 Immunization protocol**

| Group | Strain | Antigen | Adjuvant | Dose | Route^{∗} |
|---|---|---|---|---|---|
| **1** | BALB/c | PBS | None | | |
| **2** | BALB/c | PD-L1-mFc | Freund's Adjuvant, Complete (First time) | 50 µg | i.m. |
| | | | Freund's Adjuvant, Inomplete | 25 µg | |
| **3** | SJL | PBS | None | | |
| **4** | SJL | PD-L1-mFc | Freund's Adjuvant, Complete (First time) | 50 µg | i.m. |
| | | | Freund's Adjuvant, Inomplete | 25 µg | |
| **5** | SD | PBS | None | | |
| **6** | SD | PD-L1-mFc | Freund's Adjuvant, Complete (First time) | 100 µg | i.m. |
| | | | Freund's Adjuvant, Inomplete | 50 µg | |

| | | | | | |
|---|---|---|---|---|---|
| ^{∗} i.m.: intramuscular injection | | | | | |

### 2. Cell fusion

The mouse spleen was aseptically removed and prepared into a cell suspension, and the cells were fused at the ratio of spleen cells: Sp2/0 cells =1:1. The fused cell suspension was transferred to 15 mL RPMI 1640 complete medium containing 20% FBS and then left at room temperature for 20 min. The fused cells were resuspended with RPMI 1640 medium containing 1×HAT, 1×BIOMYC3, and 20% FBS. The cell suspension was added to several 96-well cell culture plates at 100 µl/well to ensure that the cell volume per well was about 4×10⁴ cells/well, and the plates was placed in a 37°C cell incubator. After 5 days, additional 100 µL of RPMI 1640 complete medium containing 20% FBS, 1×HAT, and 1×BIOMYC-3 was added to each well.

### 3. Screening of positive clones

After one week of fusion, the cell supernatant was collected. The hybridoma parent clones with binding and blocking abilities were screened by ELISA, expanded, tested for binding and blocking activities, and screened again for hybridoma positive cell lines with binding and blocking abilities. The positive cell lines were subcloned by the limiting dilution method, and after one week of culture, the activities of binding to PD-L1 molecules and blocking the interaction of PD-L1/PD-1 of the supernatant were detected by ELISA. Four preferable cell strains that showed positive results in the above two tests, PL-7, The PL-15, PL-16 and PL-18, were selected.

### 4. Acquisition of variable region sequences of anti-PD-L1 antibodies

The subcloned positive hybridoma cells were expanded, and an appropriate amount of cells was used for total RNA extraction according to the instructions of RNeasy Plus Mini Kit (Qiagen, 74134). The first strand of cDNA was synthesized using Prime Script 1st strand cDNA Synthesis Kit (Takara, 6110A).

Specific primers were designed according to the variable region of the mouse antibody subtype, and 5' end of the primers contained the homologous arm sequence for homologous recombination with the eukaryotic expression vector. PCR amplification for the variable region of antibodies was performed using cDNA as a template to obtain the gene fragments of the light chain variable region and heavy chain variable region of the mouse antibody, named SHS009PL-7, SHS009PL-15, SHS009PL-16 and SHS009PL-18, respectively. The design of primers refers to references: Anke Krebber, Susanne Bornhauser, Jorg Burmester et al. Reliable cloning of functional antibody variable domains from hybridomas and spleen cell repertoires employing a reengineered phage display system. Journal of Immunological Methods, 1997, 201: 35-55; 2. Simon KorenMihaKosmačAnjaColjaVenturinietal. Antibody variable-region sequencing as a method for hybridoma cell-line authentication, 2008, 78: 1071-1078. DNA sequencing was performed and the results are shown in Table 11.

**Table 11 Sequence table of anti-PD-L1 murine-derived monoclonal antibody**

| Antibody | Amino acid sequence of VH | Amino acid sequence of VL | H2CDR1/H2CDR2/ H2CDR3 | L2CDR1/L2CDR2/ L2CDR3 |
|---|---|---|---|---|
| SHS009PL-7 | SEQ ID NO: 99 | SEQ ID NO: 103 | SEQ ID NOs: 75, 76 and 77 | SEQ ID NOs: 78, 79 and 80 |
| SHS009PL-15 | SEQ ID NO: 100 | SEQ ID NO: 104 | SEQ ID NOs: 81, 82 and 83 | SEQ ID NOs: 84, 85 and 86 |
| SHS009PL-16 | SEQ ID NO: 101 | SEQ ID NO: 105 | SEQ ID NOs: 87, 88 and 89 | SEQ ID NOs: 90, 91 and 92 |
| SHS009PL-18 | SEQ ID NO: 102 | SEQ ID NO: 106 | SEQ ID NOs: 93, 94 and 95 | SEQ ID NOs: 96, 97 and 98 |

### 5. Construction of chimeric antibodies

The purified gene fragments of the light chain and the heavy chain variable regions of the mouse antibodies were respectively co-transformed into *Escherichia coli* DH5α competent cells with the linearized eukaryotic expression plasmid containing the light chain constant region or the heavy chain constant region of human antibodies. The chimeric antibodies with correct sequences were selected and named PL-7CHI, PL-15CHI, PL-16CHI and PL-18CHI. The sequencing results of the chimeric antibodies are shown in Table 12.

The amino acid sequences of VL and VH of PL-7CHI, PL-15CHI, PL-16CHI and PL-18CHI are identical to those of the murine-derived antibodies SHS009PL-7, SHS009PL-15, SHS009PL-16 and SHS009PL-18, respectively.

The plasmids for light and heavy chains of chimeric antibodies were transfected into HEK 293F cells, and the antibodies were expressed and purified. The purity, activity and affinity were tested and analyzed.

**Table 12 Sequence table of anti-PD-L1 chimeric antibodies**

| Chimeric antibody | Amino acid sequence of VH | Amino acid sequence of VL |
|---|---|---|
| PL-7CHI | SEQ ID NO: 99 | SEQ ID NO: 103 |
| PL-15CHI | SEQ ID NO: 100 | SEQ ID NO: 104 |
| PL-16CHI | SEQ ID NO: 101 | SEQ ID NO: 105 |
| PL-18CHI | SEQ ID NO: 102 | SEQ ID NO: 106 |

### 6. Humanization of anti-PD-L1 antibodies

Based on the results of activity analysis and affinity KD value of chimeric antibodies, PL-7CHI and PL-16CHI were modified to humanized antibodies.

The humanization of murine-derived monoclonal chimeric antibodies PL-7CHI and PL-16CHI was carried out with reference to the classical CDR transplantation strategy. With the framework region sequence FR1-FR3 of antibodies PL-7CHI and PL-16CHI as templates, full human framework regions with similar 3D structure but low immunogenicity were screened in the human framework region library to replace FR1-FR3 sequence of PL-7CHI and PL-16CHI. After homology comparison, it was found that the FR region sequences of the heavy chain variable region of the antibodies PL-7CHI and PL-16CHI were the most similar to the human antibody germline genes M99683|IGHV4-31^{∗}02 (SEQ ID NO: 107) and X62109|IGHV1-3^{∗}01 (SEQ ID NO: 108), respectively; the FR region sequences of the light chain variable region of the antibodies PL-7CHI and PL-16CHI were the most similar to the human antibody germline gene Z00023|IGKV4-1^{∗}01 (SEQ ID NO: 109). The full-length sequences of the humanized heavy/light chain were 3D modeled and compared structurally with the heavy/light chain sequences of the original antibodies. The antigenicity and 3D structural similarity were considered comprehensively, and the amino acids that were shown to play a key role in the structural stability of the antibody in the structural simulation were back mutated to murine amino acid residues. Finally, 5 humanized heavy chains (the humanized heavy chain variable region sequences of PL-7CHI: VH1-0 (SEQ ID NO: 110), VH1-1 (SEQ ID NO: 111), VH1-2 (SEQ ID NO: 112), VH1-3 (SEQ ID NO: 113) or VH1-4 (SEQ ID NO: 114)) and 4 humanized light chains (the humanized light chain variable region sequences of PL-7CHI: VL1-0 (SEQ ID NO:115), VL1-1 (SEQ ID NO:116), VL1-2 (SEQ ID NO:117) or VL1-3 (SEQ ID NO:118)) of PL-7CHI were obtained; and 5 humanized heavy chains (the humanized heavy chain variable region sequences of PL-16CHI: VH1-0 (SEQ ID NO: 119), VH1-1 (SEQ ID NO: 120), VH1-2 (SEQ ID NO: 121), VH1-3 (SEQ ID NO: 122) or VH1-4 (SEQ ID NO: 123)) and 3 humanized light chains (the humanized light chain variable region sequences of PL-16CHI: VL1-0 (SEQ ID NO: 124), VL1-1 (SEQ ID NO: 125) or VL1-2 (SEQ ID NO: 126)) of PL-16CHI were obtained. On this basis, multiple humanized antibodies were obtained through different combinations of light and heavy chains. After activity detection, it was determined that the anti-PD-L1 humanized antibodies with the highest scores were HuPL7-21 and HuPL16-42.

### Example 13 Production of humanized anti-PD-L1 antibodies

Corresponding polynucleotides were synthesized based on the amino acid sequences of the light chain and heavy chain variable region of humanized antibody obtained above, and oligonucleotide fragments containing complementary sequences between adjacent fragments were synthesized. The oligonucleotide fragments were annealed and assembled by Overlap PCR. Then nucleotide fragments encoding the entire light chain and heavy chain variable regions were amplified by specific primers (5' end contained the homologous arm sequence for homologous recombination with the eukaryotic expression vector). The purified nucleotide fragments of the light chain variable region were co-transformed into *Escherichia coli* DH5α competent cells with the linearized eukaryotic expression plasmid containing light chain constant region of IgG4. The purified nucleotide fragments of the heavy chain variable region were co-transformed into *Escherichia coli* DH5α competent cells with the eukaryotic expression plasmid containing heavy chain constant region of IgG4 containing S228P/L235E mutation. The competent cells with the transformed plasmids were spread evenly on the surface of the agar plates containing the corresponding antibiotics. The agar plates were incubated in a 37°C constant temperature incubator overnight, and then several single colonies were picked out for DNA sequencing.

The positive clones with correct sequences were subjected to plasmid extraction to obtain the expression plasmids for the light chain and the heavy chain of humanized antibodies. The concentration and purity of the plasmids were detected by a nucleic acid quantitative analyzer.

The plasmids were transfected into HEK293 F cells, and a large number of antibodies were expressed and purified. The purity, activity and affinity were tested and analyzed. The sequences are shown in Table 13.

**Table 13 Sequence table of anti-PD-L1 humanized antibodies**

| Humanized antibody | Amino acid sequence | | Nucleotide sequence | |
|---|---|---|---|---|
| | VH | VL | VH | VL |
| HuPL7-21 | SEQ ID NO: 112 | SEQ ID NO: 116 | SEQ ID NO: 127 | SEQ ID NO: 129 |
| HuPL16-42 | SEQ ID NO: 123 | SEQ ID NO: 126 | SEQ ID NO: 128 | SEQ ID NO: 130 |

For the antibody HuPL7-21, the sequences of CDR1, CDR2 and CDR3 of VH are set forth in SEQ ID NOs: 75, 76 and 77, respectively; the sequences of CDR1, CDR2 and CDR3 of VL are set forth in SEQ ID NOs: 78 and 79 and 80, respectively. For the antibody HuPL16-42, the sequences of CDR1, CDR2 and CDR3 of VH are set forth in SEQ ID NOs: 87, 88 and 89, respectively; the sequences of CDR1, CDR2 and CDR3 of VL are set forth in SEQ ID NOs: 90, 91 and 92, respectively.

### Example 14 Construction, expression and purification of anti-CD47/PD-L1 bispecific antibodies

### 1. Construction of expression vector for bispecific anti-CD47/anti-PD-L1 antibodies

The anti-CD47/anti-PD-L1 bispecific antibodies were constructed by genetic engineering, and the structure of antibodies is shown in FIG. 8. The bispecific antibody is formed by heterodimerization of two single chains from anti-PD-L1 antibody and anti-CD47 antibody, respectively. Unlike the natural IgG antibody, the light chains of both anti-PD-L1 and anti-CD47 antibodies in this bispecific antibody are linked to the N-terminal of the heavy chain by an additional flexible linking peptide, which is a GGGGS repeat sequence containing glycine (G) and serine (S) residues, preferably a sequence containing eight GGGGS repeats. In addition, in order to promote the formation of heterodimers, the S354C/T366W mutation is further added to the CH3 domain of the anti-PD-L1 antibody single chain and the Y349C/T366S/L368A/Y407V mutation to the CH3 domain of the anti-CD47 antibody single chain on the basis of the above S228P/L235E mutation. According to this structural form, the sequences of the above-mentioned anti-PD-L1 antibody HuPL7-21 (light chain variable region set forth in SEQ ID NO: 116 and constant region CL set forth in SEQ ID NO: 131) or HuPL16-42 (light chain variable region set forth in SEQ ID NO: 126 and constant region CL set forth in SEQ ID NO: 131) were used to obtain the nucleotide fragment of the anti-PD-L1 antibody single chain by gene synthesis, namely ScFabHuPL7-21Ks or ScFabHuPL16-42Ks. The sequences of the above-mentioned anti-CD47 antibody Hu34-39 (light chain variable region set forth in SEQ ID NO: 33 and constant region CL set forth in SEQ ID NO: 131) were used to obtain the nucleotide fragment of the anti-CD47 antibody single chain by gene synthesis, namely ScFabHu34-39Hs. The nucleotide fragments ScFabHuPL7-21Ks (or ScFabHuPL16-42Ks) and ScFabHu34-39Hs (upstream and downstream containing homology arms of appropriate length) were respectively co-transformed into *Escherichia coli* DH5α competent cells with the linearized eukaryotic expression plasmid pHR. The competent cells transformed with the plasmids were spread evenly on the surface of the agar plates containing the corresponding antibiotics. The agar plates were incubated in a 37°C constant temperature incubator overnight, and then several single colonies were picked out for DNA sequencing. The positive clones with correct sequence were subjected to plasmid extraction to obtain ScFabHuPL7-21Ks (or ScFabHuPL16-42Ks) and ScFabHu34-39Hs expression vectors.

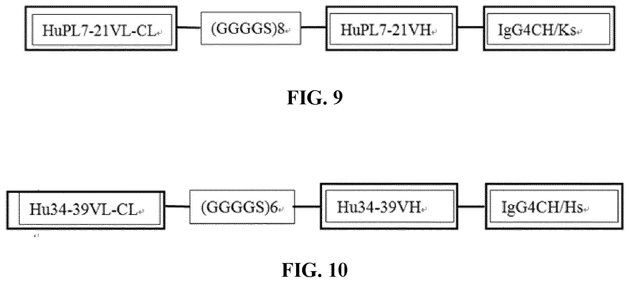

FIG. 9 shows a schematic diagram of the sequence structure of ScFabHuPL7-21Ks, wherein HuPL7-21VL-CL represents humanized PD-L1 monoclonal antibody HuPL7-21 light chain; (GGGGS)8 represents a flexible linking peptide of 8 GGGGS repeats; HuPL7-21VH represents humanized PD-L1 monoclonal antibody HuPL7-21 heavy chain variable region; IgG4CH/Ks represents IgG4 heavy chain constant region (specific sequence set forth in, for example, SEQ ID NO: 132) containing S228P/L235E/S354C/T366W mutation to form a "Knob" structure.

FIG. 10 shows a schematic diagram of the sequence structure of ScFabHu34-39Hs, wherein Hu34-39VL-CL represents humanized CD47 monoclonal antibody Hu34-39 light chain; (GGGGS)6 represents a flexible linking peptide of 6 GGGGS repeats; Hu34-39VH represents humanized CD47 monoclonal antibody Hu34-39 heavy chain variable region; IgG4CH/Hs represents IgG4 heavy chain constant region (specific sequence set forth in, for example, SEQ ID NO: 133) containing S228P/L235E/Y349C/T366S/L368A/Y407V mutation to form a "Hole" structure.

The CL sequence in the above structure is set forth in SEQ ID NO: 131.

### 2. Transient expression of anti-CD47/anti-PD-L1 bispecific antibody in Expi-CHO cells

Expi-CHO cells were transfected with the recombinant plasmids of the above two expression vectors ScFabHuPL7-21Ks (or ScFabHuPL16-42Ks) and ScFabHu34-39Hs using the Expi-Fectamine CHO Transfection Kit. After culturing in serum-free medium for 14 days, the supernatant of Expi-CHO cells was collected and detected the expression of the bispecific antibody by Western blotting. The bispecific antibody formed by dimerization of two single chains of ScFabHuPL7-21Ks and ScFabHu34-39Hs was named ScFab (HuPL7-21Ks/Hu34-39Hs), and the bispecific antibody formed by dimerization of two single chains of ScFabHuPL16-42Ks and ScFabHu34-39Hs was named ScFab (HuPL16-42Ks/Hu34-39Hs).

### 3. Purification of bispecific anti-CD47/anti-PD-L1 antibodies

After expressed and secreted in Expi-CHO cells, the bispecific antibodies of the present disclosure were purified by the method of Protein A affinity chromatography with the following specific steps. After the Protein A affinity chromatography column was equilibrated with buffer, the supernatant of Expi-CHO cell culture concentrated by ultrafilter was injected, monitored at A280 (nm), washed with washing solution until the unbound protein was all washed away, and then the antibodies were eluted with elution buffer to obtain the corresponding bispecific antibodies. The purified bispecific antibodies were tested for purity by SEC-HPLC and for molecular weight by LC-MS, and subjected to quality identification for subsequent pharmaceutical research. The identification results of SEC-HPLC and LC-MS showed that the purities of the bispecific antibodies ScFab (HuPL7-21Ks/Hu34-39Hs) and ScFab (HuPL16-42Ks/Hu34-39Hs) both reached more than 95%, and the determination value of molecular weight matched the theoretical value.

### Example 15 Construction of stable cell line with high expression of hPD-L1

### Construction of stable cell lines CHO-K1-hPD-L1 and Raji-hPD-L1 with high expression of hPD-L1

CHO-K1 cells (from Shanghai Institute of Biochemistry and Cell Biology, Chinese Academy of Sciences) and Raji cells (from ImmuneOnco Biopharmaceuticals Inc.) were transfected with the eukaryotic expression plasmid pTargeT-hPD-L1 containing hPD-L1 (human PD-L1) extracellular region sequence (UniProtKB-Q9NZQ7(PD-L1 Human)>sp|Q9NZQ7|19-238, SEQ ID NO: 134) by electroporation, and cultured in an incubator at 37°C and 5% CO₂. After 24 h, cells were selected with a medium containing 500 µg/ml G418. After 12 days, the positive rate of the cell pool was detected by FACS. The cells after electroporation were plated (at a cell density of 1×10⁶ cells /ml, 100 µl /well) and incubated with FITC anti-human PD-L1 antibody (SINO BIOLOGICAL, 10084-MMB6-F) at 4°C for 60 min. The mean value under the FITC channel was read by a flow cytometer. After data analysis, the positive cell lines were selected for subcloning, and the CHO-K1/Raji cells from a single clone were selected. The cell lines with high expression of PD-L1 were named CHO-K1-hPD-L1 and Raji-hPD-L1.

### Example 16 In vitro binding and blocking tests of anti-CD47/anti-PD-Ll bispecific antibody (by ELISA)

### 1. Anti-CD47/anti-PD-L1 bispecific antibody binding to PD-L1 (by ELISA)

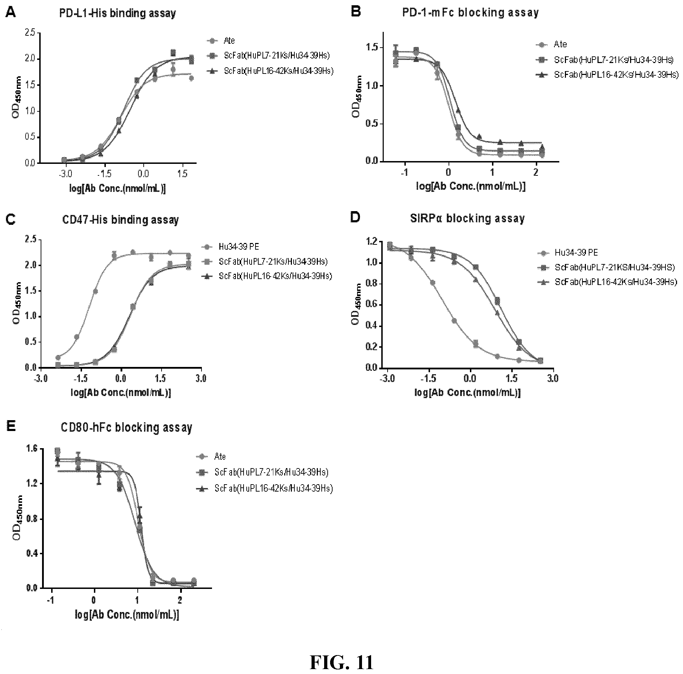

Human PD-L1-His protein (0.5 µg/ml, 100 µl/well) was coated on a 96-well ELISA plate and incubated at 37°C for 2 h. After washing 3 times with 1×PBST, the plate was blocked with 5% nonfat milk at 4°C overnight and then washed 3 times with 1×PBST. The bispecific antibody was added to the ELISA plate with concentrations starting from 10 µg /mL at 5-fold serial dilution, and incubated at 37°C for 1.5 h, with Atezolizumab (Sino Biological, Cat: 68049-H001, abbreviated Ate) as a control antibody. After washing 5 times with 1×PBST, HRP-Anti-Human IgG secondary antibody (Jackson, 109-035-003, 1:10000) was added and incubated at 37°C for 40 min. After washing 5 times with 1×PBST, color developing solution TMB was added to the plate, and a microplate reader (Thermo, Multiskan FC) was used to read OD450 value after termination of the reaction. The EC₅₀ results are shown in FIG. 11.

The experimental results show that both the bispecific antibodies ScFab (HuPL7-21Ks/Hu34-39Hs) and ScFab (HuPL16-42Ks/Hu34-39Hs) are able to bind to human PD-L1, and the binding to human PD-L1 was comparable to that of Atezolizumab.

### 2. Anti-CD47/anti-PD-L1 bispecific antibody competing with PD-1 (by ELISA)

The activity of antibodies to block the binding of PD1 to PD-L1 was analyzed by ELISA. Human PD-L1-hFC protein (2 µg/ml, 100 µl/well) was coated on a 96-well ELISA plate and incubated at 37°C for 2 h. After washing 3 times with 1×PBST, the plate was blocked with 5% nonfat milk at 4°C overnight and then washed 3 times with 1×PBST. The bispecific antibody ScFab (HuPL7-21Ks/Hu34-39Hs) or ScFab (HuPL16-42Ks/Hu34-39Hs) as the primary antibody was added to the ELISA plate with a total of 8 concentrations starting from 10 µg /mL at 3-fold serial dilution, and incubated together with 1 µg/mL PD-1-mFc at 37°C for 1.5 h in the presence of serially diluted anti-PD-L1 antibody, with Atezolizumab (Sino Biological, Cat: 68049-H001, abbreviated Ate) as a control antibody. After washing 5 times with 1×PBST, Anti-Mouse IgG HRP (Jackson, 109-035-003, 1:10000) was used as the secondary antibody and incubated at 37°C for 1 h. After washing 5 times with 1×PBST,color developing solution TMB was added to the plate, and the microplate reader was used to read OD450 value after termination of the reaction. The results are shown in FIG. 11.

The experimental results show that both the bispecific antibodies ScFab (HuPL7-21Ks/Hu34-39Hs) and ScFab (HuPL16-42Ks/Hu34-39Hs) had the ability to block the binding of PD-L1 to PD-1, and the blocking ability was comparable to that of Atezolizumab.

### 3. Anti-CD47/anti-PD-L1 bispecific antibody binding to CD47 (by ELISA)

The binding activity of antibodies to CD47 was analyzed by ELISA. Human CD47-His protein (0.5 µg/ml, 100 µl/well) was coated on a 96-well ELISA plate and incubated at 37°C for 2 h. After washing 3 times with 1×PBST,the plate was blocked with 5% nonfat milk at 4°C overnight and then washed 3 times with 1×PBST. The bispecific anti-CD47/anti-PD-L1 antibody provided by the present disclosure was added to the ELISA plate with a total of 8 concentrations starting from 50 µg /mL at 5-fold serial dilution, and incubated at 37°C for 1.5 h, with Hu34-39-PE as a control antibody. After washing 5 times with 1×PBST, HRP-Anti-Human IgG (1:10000) was used as the secondary antibody and incubated at 37°C for 40 min. After washing 5 times with 1×PBST, color developing solution TMB was added to the plate, and a microplate reader (Thermo, Multiskan FC) was used to read OD450 value after termination of the reaction. The EC₅₀ results are shown in FIG. 11.

The experimental results show that both the bispecific antibodies ScFab (HuPL7-21Ks/Hu34-39Hs) and ScFab (HuPL16-42Ks/Hu34-39Hs) are able to bind to human CD47, and their binding activities were reduced to varying degrees. The binding EC₅₀ value of ScFab (HuPL7-21Ks/Hu34-39Hs) was 40-fold higher than that of Hu34-39-PE.

### 4. Anti-CD47/anti-PD-L1 bispecific antibody competing with SIRPα (by ELISA)

The blocking activity of antibodies was analyzed by ELISA. Human CD47-His protein (0.4 µg/ml, 100 µl/well) was coated on a 96-well ELISA plate and incubated at 37°C for 2 h. After washing 3 times with 1×PBST,the plate was blocked with 5% nonfat milk at 4°C overnight and then washed 3 times with 1×PBST. The bispecific antibody ScFab (HuPL7-21Ks/Hu34-39Hs) or ScFab (HuPL16-42Ks/Hu34-39Hs) as the primary antibody was added to the ELISA plate with a total of 8 concentrations starting from 10 µg /mL at 3-fold serial dilution, and incubated together with 2 µg/mL SIRPα-biotin at 37°C for 1.5 h in the presence of serially diluted anti-CD47 antibody, with Hu34-39-PE as a control antibody. After washing 5 times with 1×PBST,SA-HRP (Jackson, 109-035-003, 1:10000) was used as the secondary antibody and incubated at 37°C for 1 h. After washing 5 times with 1×PBST, color developing solution TMB was added to the plate, and the microplate reader was used to read OD450 value after termination of the reaction. The results are shown in FIG. 11.

The experimental results show that both the bispecific antibodies ScFab (HuPL7-21Ks/Hu34-39Hs) and ScFab (HuPL16-42Ks/Hu34-39Hs) are able to block the binding of CD47 to SIRPα, and the blocking ability was lower than that of Hu34-39-PE, with an IC₅₀ value increased by about 40 times.

### 5. Anti-CD47/anti-PD-L1 bispecific antibody competing with CD80 (by ELISA)

The activity of antibodies to block the binding of PD-L1 to CD80 was analyzed by ELISA. CD80-hFc protein (8 µg/ml, 100 µl/well) was coated on a 96-well ELISA plate and incubated overnight. After washing 3 times with 1×PBST, the plate was blocked with 5% nonfat milk at 37°C for 2 h and then washed 3 times with 1×PBST. The bispecific antibody ScFab (HuPL7-21Ks/Hu34-39Hs) or ScFab (HuPL16-42Ks/Hu34-39Hs) as the primary antibody was added to the ELISA plate with a total of 8 concentrations starting from 30 µg /mL at 3-fold serial dilution, and incubated together with PD-L1-mFc at 37°C for 1.5 h in the presence of serially diluted anti-PD-L1 antibody, with Atezolizumab as a control antibody. After washing 5 times with 1×PBST, Anti-Mouse IgG HRP was used as the secondary antibody and incubated at 37°C for 1 h. After washing 5 times with 1×PBST, color developing solution TMB was added to the plate, and the microplate reader was used to read OD450 value after termination of the reaction. The results are shown in FIG. 11

The experimental results show that both the bispecific antibodies ScFab (HuPL7-21Ks/Hu34-39Hs) and ScFab (HuPL16-42Ks/Hu34-39Hs) are able to block the binding of PD-L1 to CD80, and the blocking ability was comparable to that of Atezolizumab.

The above experimental results show that the bispecific antibodies of the present disclosure bind to PD-L1/CD47 with different activities, so as to reduce the toxic response of the antibodies such as blood toxicity while ensuring the anti-tumor activity.

### Example 17 Binding/blocking test of anti-CD47/anti-PD-Ll bispecific antibody at cellular level

### 1. Binding activity of bispecific antibodies ScFab (HuPL7-21Ks/Hu34-39Hs) and ScFab (HuPL16-42Ks/Hu34-39Hs) to PD-L1/CD47 on cell surface detected by FACS

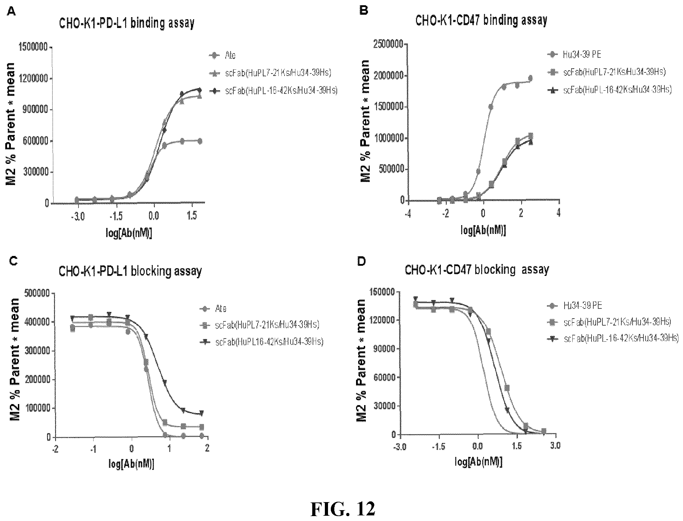

The CHO-K1-hPD-L1/CHO-K1-hCD47 stable cell line was used as a PD-L1/CD47 provider, and the serially diluted bispecific anti-PD-L1/anti-CD47 antibody was added to the cells as the primary antibody and incubated at 4°C for 1.5 h. PE Anti-Human IgG was used as the secondary antibody and incubated at 4°C for 1 h. Atezolizumab and Hu34-39-PE were used as positive controls. The product of the mean value and the Parent value at a wavelength of 585 nm was read using a flow cytometer, and the results are shown in FIG. 12.

The experimental results show that both the bispecific antibodies ScFab (HuPL7-21Ks/Hu34-39Hs) and ScFab (HuPL16-42Ks/Hu34-39Hs) are able to bind to human PD-L1 on cell surface, and the binding ability was comparable to that of Atezolizumab. The binding activity of bispecific antibodies ScFab (HuPL7-21Ks/Hu34-39Hs) and ScFab (HuPL16-42Ks/Hu34-39Hs) to CD47 on cell surface was lower than that of Hu34-39-PE monoclonal antibody, with EC₅₀ increased by about 4 times and Emax decreased by about

### 2 times.

### 2. Ability of bispecific antibodies ScFab (HuPL7-21Ks/Hu34-39Hs) and ScFab (HuPL16-42Ks/Hu34-39Hs) to block the binding of PD-1/SIRPα to cell surface detected by FACS

The CHO-K1-hPD-L1/CHO-K1-hCD47 stable cell line was used as a PD-L1/CD47 provider to observe the binding of PD-L1 to PD-1 and the binding of CD47 to SIRPα in the presence of serially diluted anti-PD-L1 antibody/anti-CD47 antibody. The bispecific antibody ScFab (HuPL7-21Ks/Hu34-39Hs) or ScFab (HuPL16-42Ks/Hu34-39Hs) was used as the primary antibody, added to the cells after serial dilution together with 1 µg /mL PD-1-mFc and SIRPα-biotin respectively, and incubated at 37°C for 1.5 h. PE-Anti-Mouse IgG/PE-SA was used as the secondary antibody. Atezolizumab was used as a positive control for blocking the binding of PD-1-mFc to PD-L1 on cell surface, and Hu34-39-PE was used as a positive control for blocking the binding of SIRPα to CD47 on cell surface. The results are shown in FIG. 12.

The experimental results show that both the bispecific antibodies ScFab (HuPL7-21Ks/Hu34-39Hs) and ScFab (HuPL16-42Ks/Hu34-39Hs) are able to block the binding of human PD-1 to CHO-K1-PD-L1 and SIRPα to CHO-K1-CD47, and the ability to block the binding of PD-1 to CHO-K1-PD-L1 was comparable to that of Atezolizumab. The ability of the bispecific antibodies to block the binding of SIRPα to CHO-K1-CD47 was lower than that of Hu34-39-PE, with an IC₅₀ value increased by 3 times.

According to the results of ELISA and FACS, the bispecific antibodies ScFab (HuPL7-21Ks/Hu34-39Hs) and ScFab (HuPL16-42Ks/Hu34-39Hs) had the similar ability of binding to and blocking of PD-L1 as Atezolizumab. While the ability of binding to CD47 decreased, the EC₅₀ increased by about 40 times detected by ELISA and the EC₅₀ increased by about 4 times and Emax decreased by 2 times detected by FACS. That is, the bispecific antibodies ScFab (HuPL7-21Ks/Hu34-39Hs) and ScFab (HuPL16-42Ks/Hu34-39Hs) bind to PD-L1/CD47 with different activities, which help to enhance the tumor targeting and reduce the adverse reactions, especially those against red blood cells, of the bispecific antibodies.

### Example 18 Double binding and double blocking tests of anti-CD47/anti-PD-Ll bispecific antibody on Raji-hPD-Ll cells detected by FACS

Raji-hPD-L1 tumor cells were purchased from ImmuneOnco Biopharmaceuticals Inc.. Both of hPD-L1and hCD47 were highly expressed on the surface of Raji-hPD-L1 cells. The bispecific antibodies ScFab (HuPL7-21Ks/Hu34-39Hs) and ScFab (HuPL16-42Ks/Hu34-39Hs) bound to both hPD-L1and hCD47 on the surface of Raji-hPD-L1 cells, and also blocked the binding of CD47/SIRPα and the binding of PD-1/PD-L1 at the same time, showing dual-arm binding and dual-arm blocking activities. Dual-blocking: 2.4×10⁵ per well Raji-hPD-L1 cells were plated in a plate with U-bottom. For the addition of the primary antibody, the antibody was diluted in 2-fold serial dilution to a total of 9 working concentrations starting from 2.5 µg /mL; SIRPα-mFc with a final antigen concentration of 1 µg /mL was evenly mixed with PD-1-mFc with a final concentration of 1 µg/mL; and 50 µl of the antibody and 50 µl of the antigen were premixed in each well, added to cell wells with a total of 100 µl, and then incubated at 4°C for 1.5 h. For the addition of the secondary antibody, 200 µl of cell stain buffer was added first to wash three times, and 0.8 µl of PE-anti-mouse-IgG-Fc was added to each well and incubated at 4°C for 1 h. Finally, 200 µl of cell stain buffer was added to wash three times, and 100 µl of cell stain buffer was added to resuspend the cells. A flow cytometry was used for detection. Dual-binding: 1.5×10⁶ per well Raji-hPD-L1 cells were plated in a plate with U-bottom. For the addition of the primary antibody, the antibody was diluted in 5-fold serial dilution to a total of 8 working concentrations starting from 10 µg /mL, and then incubated at 4°C for 1.5 h. For the addition of the secondary antibody, 200 µl of cell stain buffer was added first to wash three times, and 0.8 µl of PE-anti-human-IgG-Fc was added to each well and incubated at 4°C for 1 h. Finally, 200 µl of cell stain buffer was added to wash three times, and 100 µl of cell stain buffer was added to resuspend the cells. A flow cytometry was used for detection.

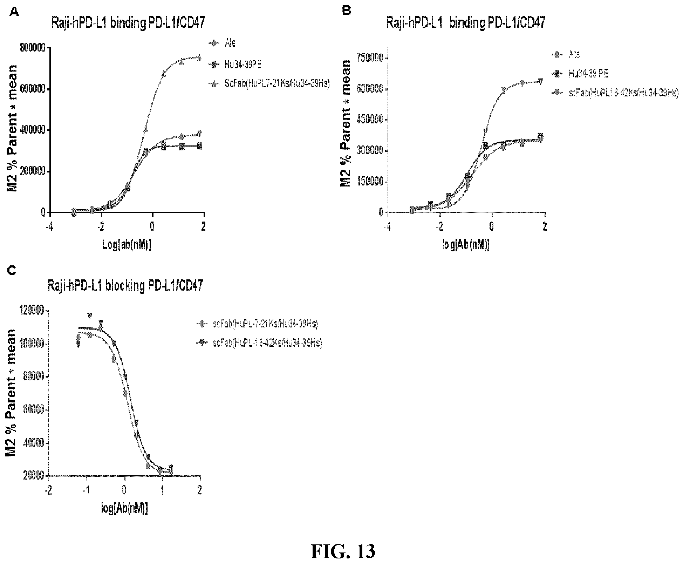

The experimental results are shown in FIG. 13. The results show that the bispecific antibody of the present disclosure bound to cells with dual-target, and simultaneously blocked the binding of CD47/SIRPα and the binding of PD-1/PD-L1 on the surface of the cells.

### Example 19 Detection of the inhibition of anti-CD47/anti-PD-Ll bispecific antibody on the growth of transplanted tumor in mice

With the characteristics of NOD, Prkdcscid, IL2rgnull deletion/mutation, NSG mice are the tool mice with the highest degree of immunodeficiency and are most suitable for human cell transplantation, showing little rejection of human cells and tissues. In the present disclosure, NSG mice (purchased from Biocytogen Pharmaceuticals (Beijing) Co., Ltd.) and Raji-hPD-L1 tumor cells were used to establish a tumor xenograft model, Raji-PBMC-NSG model, to study the antitumor effect of the anti-CD47/anti-PD-L1 bispecific antibody ScFab (HuPL7-21Ks/Hu34-39Hs) in a subcutaneous transplant model of Raji-hPD-L1 lymphoma. The anti-CD47 monoclonal antibody 5F9 was purchased from Sino Biological Inc. (Cat: 68063-H001). Table 14 shows the experimental design of the antitumor effect of the test drugs in Raji-PBMC-NSG tumor model.

**Table 14 Experimental design for drug test in Raji-PBMC-NSG model**

| Group | N | Antibody | Mouse strain | Dosage (mg/kg) | Dosing volume (µl/g) | Route of administration | Dosing frequency |
|---|---|---|---|---|---|---|---|
| 1 | 6 | PBS | NSG | --- | 10 | i.v. | q.w×3 |
| 2 | 6 | Atezolizumab | NSG | 10 | 10 | i.v. | q.w×3 |
| 3 | 6 | Atezolizumab+5F9 | NSG | 10+10 | 10 | i.v. | q.w×3 |
| 4 | 6 | BiAb | NSG | 10 | 10 | i.v. | q.w×3 |
| 5 | 6 | BiAb | NSG | 20 | 10 | i.v. | q.w×3 |

| | | | | | | | |
|---|---|---|---|---|---|---|---|
| a: N refers to the number of mice per group; b: BiAb refers to bispecific antibody ScFab (HuPL7-21Ks/Hu34-39Hs). | | | | | | | |

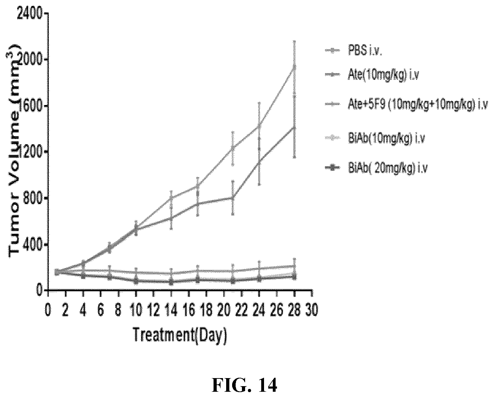

The changes of tumor volume in each group over time are shown in FIG. 14. The anti-tumor effect of the bispecific antibody of the present disclosure was significantly superior to that of Atezolizumab, and was better than the combination of monoclonal antibodies (Atezolizumab+5F9).

### Example 20 Acute toxicity test of anti-CD47/anti-PD-Ll bispecific antibody in cynomolgus monkey

In this example, a toxicity test of the anti-CD47/anti-PD-L1 bispecific antibody ScFab (HuPL7-21Ks/Hu34-39Hs) of the present disclosure (abbreviated as BiAb) was performed by administering to cynomolgus monkeys by single intravenous infusion. The administration doses of the bispecific antibody were 10, 30 and 100 mg/kg, one male and one female cynomolgus monkeys in each group. The anti-CD47 monoclonal antibody Hu34-39-PE was administered at a dose of 30 mg/kg, and Hu5F9 was administered at a dose of 20 mg/kg, with two cynomolgus monkeys in each group.

Monkeys were administered by single intravenous infusion, with an observation period of 21 days. Blood samples were collected from the femoral vein at different time points for the detection of blood cell count, coagulation function indexes, and blood biochemical indexes.

The results of drug safety evaluation show that as of the 21st day, no monkeys died in all groups, and there were no abnormalities in general state observation, food intake, body weight, etc. in each group.

Animals given the bispecific antibody ScFab (HuPL7-21Ks/Hu34-39Hs) showed no change in RBC count, HGB content and RET%. The bispecific antibody ScFab (HuPL7-21Ks/Hu34-39Hs) did not show toxicity on red blood cells and other hematological toxicity at doses of 10, 30 and 100 mg/kg.

Animals given the monoclonal antibody Hu5F9 (20 mg/kg) showed a significant decrease in RBC count and HGB content, and a significant increase in RET%. Animals given the monoclonal antibody Hu34-39-PE (30 mg/kg) showed a certain extent of decrease in RBC count and HGB content, and an increase in RET% less than the monoclonal antibody Hu5F9. Hu5F9 showed obvious toxicity on red blood cells, and Hu34-39-PE showed a weaker toxicity on red blood cells than Hu5F9. The safety of the bispecific antibody ScFab (HuPL7-21Ks/Hu34-39Hs) for red blood cells was significantly better than that of monoclonal antibodies Hu34-39-PE and Hu5F9.

The above examples show that the anti-CD47/anti-PD-L1 bispecific antibody ScFab (HuPL7-21Ks/Hu34-39Hs) of the present disclosure differentially binds to CD47 and PD-L1, and fairly retains the activities of binding to and blocking of PD-L1, without toxicity on red blood cells and other hematological toxicity, exhibiting an excellent safety.

### Example 21 Detection of the inhibition of anti-PD-Ll antibody on the growth of transplanted tumor in mice

In the present disclosure, NSG mice (purchased from Biocytogen Pharmaceuticals (Beijing) Co., Ltd., China) and Raji-hPD-L1 tumor cells (purchased from ImmuneOnco Biopharmaceuticals Inc., China) were used to establish a tumor xenograft model, Raji-PBMC-NSG model, to study the antitumor effect of the antibody of the present disclosure in a subcutaneous transplant model of Raji-hPD-L1 lymphoma. The anti-PD-L1 positive control antibody was Atezolizumab (Sino Biological, Cat: 68049-H001). There were 6 mice in each group. The negative control group was given normal saline (PBS). The anti-PD-L1 antibody HuPL7-21 of the present disclosure and Atezolizumab were administered at a dose of 10 mg/kg, respectively. The route of administration was intraperitoneal injection, and the dosing frequency was two times a week for three consecutive weeks.

At the end of the experiment, the tumor inhibition rate (TGI_{TV}) % of each group was calculated, as shown in Table 15.

**Table 15 Effect of each antibody on the tumor volume of Raji-PBMC-NSG model mice**

| **Group** | **Dosage (mg/kg)** | **TGI_{TV} (%)** |
|---|---|---|
| PBS | - | 0.00 |
| Atezolizumab | 10 | 37.40 |
| HuPL7-21 | 10 | 48.75 |

The anti-PD-L1 antibody HuPL7-21 of the present disclosure has a significantly better anti-tumor effect *in vivo* than Atezolizumab.

## Claims

1. An anti-CD47/anti-PD-L1 antibody comprising an anti-CD47 antibody or antigen-binding fragment thereof and an anti-PD-L1 antibody or antigen-binding fragment thereof, wherein:
the anti-CD47 antibody or antigen-binding fragment thereof comprises a first heavy chain variable region and a first light chain variable region, wherein:
(1) the first heavy chain variable region comprises H1CDR1, H1CDR2 and H1CDR3 comprising amino acid sequences set forth in SEQ ID NOs: 4, 5 and 6, or amino acid sequences having at least 85% sequence identity to the amino acid sequences set forth in SEQ ID NOs: 4, 5 and 6, respectively; and
(2) the first light chain variable region comprises L1CDR1, L1CDR2 and L1CDR3 comprising amino acid sequences set forth in SEQ ID NOs: 15, 16 and 17, or amino acid sequences having at least 85% sequence identity to the amino acid sequences set forth in SEQ ID NOs: 15, 16 and 17, respectively.

2. The anti-CD47/anti-PD-L1 antibody according to claim 1, wherein the anti-PD-L1 antibody or antigen-binding fragment thereof comprises a second heavy chain variable region and a second light chain variable region, wherein:
(1) the second heavy chain variable region comprises H2CDR1, H2CDR2 and H2CDR3 selected from the group consisting of:
(A1) amino acid sequences set forth in SEQ ID NOs: 75, 76 and 77;
(A2) amino acid sequences set forth in SEQ ID NOs: 81, 82 and 83;
(A3) amino acid sequences set forth in SEQ ID NOs: 87, 88 and 89;
(A4) amino acid sequences set forth in SEQ ID NOs: 93, 94 and 95; and
(A5) amino acid sequences having at least 85% sequence identity to the amino acid sequences set forth in (A1), (A2), (A3) or (A4); and
(2) the second light chain variable region comprises L2CDR1, L2CDR2 and L2CDR3 selected from the group consisting of:
(A6) amino acid sequences set forth in SEQ ID NOs: 78, 79 and 80;
(A7) amino acid sequences set forth in SEQ ID NOs: 84, 85 and 86;
(A8) amino acid sequences set forth in SEQ ID NOs: 90, 91 and 92;
(A9) amino acid sequences set forth in SEQ ID NOs: 96, 97 and 98; and
(A10) amino acid sequences having at least 85% sequence identity to the amino acid sequences set forth in (A6), (A7), (A8) or (A9).

3. The anti-CD47/anti-PD-L1 antibody according to claim 2, wherein in the anti-PD-L1 antibody or antigen-binding fragment thereof:
the second heavy chain variable region comprises H2CDR1, H2CDR2 and H2CDR3 comprising amino acid sequences set forth in SEQ ID NOs: 75, 76 and 77, or amino acid sequences having at least 85% sequence identity to the amino acid sequences set forth in SEQ ID NOs: 75, 76 and 77, respectively, and the second light chain variable region comprises L2CDR1, L2CDR2 and L2CDR3 comprising amino acid sequences set forth in SEQ ID NOs: 78, 79 and 80, or amino acid sequences having at least 85% sequence identity to the amino acid sequences set forth in SEQ ID NOs: 78, 79 and 80, respectively;
the second heavy chain variable region comprises H2CDR1, H2CDR2 and H2CDR3 comprising amino acid sequences set forth in SEQ ID NOs: 87, 88 and 89, or amino acid sequences having at least 85% sequence identity to the amino acid sequences set forth in SEQ ID NOs: 87, 88 and 89, respectively, and the second light chain variable region comprises L2CDR1, L2CDR2 and L2CDR3 comprising amino acid sequences set forth in SEQ ID NOs: 90, 91 and 92, or amino acid sequences having at least 85% sequence identity to the amino acid sequences set forth in SEQ ID NOs: 90, 91 and 92, respectively;
the second heavy chain variable region comprises H2CDR1, H2CDR2 and H2CDR3 comprising amino acid sequences set forth in SEQ ID NOs: 81, 82 and 83, or amino acid sequences having at least 85% sequence identity to the amino acid sequences set forth in SEQ ID NOs: 81, 82 and 83, respectively, and the second light chain variable region comprises L2CDR1, L2CDR2 and L2CDR3 comprising amino acid sequences set forth in SEQ ID NOs: 84, 85 and 86, or amino acid sequences having at least 85% sequence identity to the amino acid sequences set forth in SEQ ID NOs: 84, 85 and 86, respectively; or
the second heavy chain variable region comprises H2CDR1, H2CDR2 and H2CDR3 comprising amino acid sequences set forth in SEQ ID NOs: 93, 94 and 95, or amino acid sequences having at least 85% sequence identity to the amino acid sequences set forth in SEQ ID NOs: 93, 94 and 95, respectively, and the second light chain variable region comprises L2CDR1, L2CDR2 and L2CDR3 comprising amino acid sequences set forth in SEQ ID NOs: 96, 97 and 98, or amino acid sequences having at least 85% sequence identity to the amino acid sequences set forth in SEQ ID NOs: 96, 97 and 98, respectively.

4. The anti-CD47/anti-PD-L1 antibody according to any one of claims 1-3, wherein:
(1) the first heavy chain variable region comprises an amino acid sequence selected from the group consisting of:
(b1) amino acid sequences set forth in SEQ ID NO: 22 and SEQ ID NO: 30,
(b2) amino acid sequences derived from the amino acid sequences set forth in (b1) by substitution, deletion or addition of one or more amino acids and functionally identical or similar to the amino acid sequences set forth in (b1), and
(b3) amino acid sequences having at least 80% sequence identity to the amino acid sequences set forth in (b1); and
(2) the first light chain variable region comprises an amino acid sequence selected from the group consisting of:
(b4) amino acid sequences set forth in SEQ ID NO: 25 and SEQ ID NO: 33,
(b5) amino acid sequences derived from the amino acid sequences set forth in (b4) by substitution, deletion or addition of one or more amino acids and functionally identical or similar to the amino acid sequences set forth in (b4), and
(b6) amino acid sequences having at least 80% sequence identity to the amino acid sequences set forth in (b4).

5. The anti-CD47/anti-PD-L1 antibody according to any one of claims 1-4, comprising an anti-PD-L1 antibody or antigen-binding fragment thereof, wherein:
the anti-PD-L1 antibody or antigen-binding fragment thereof comprises a second heavy chain variable region and a second light chain variable region, wherein:
(1) the second heavy chain variable region comprises an amino acid sequence selected from the group consisting of:
(B1) amino acid sequences set forth in SEQ ID NOs: 99, 100, 101, 102, 110, 111, 112, 113, 114, 119, 120, 121, 122 and 123,
(B2) amino acid sequences derived from the amino acid sequences set forth in (B1) by substitution, deletion or addition of one or more amino acids and functionally identical or similar to the amino acid sequences set forth in (B1), and
(B3) amino acid sequences having at least 80% sequence identity to the amino acid sequences set forth in (B1); and
(2) the second light chain variable region comprises an amino acid sequence selected from the group consisting of:
(B4) amino acid sequences set forth in SEQ ID NOs: 103, 104, 105, 106, 115, 116, 117, 118, 124, 125 and 126,
(B5) amino acid sequences derived from the amino acid sequences set forth in (B4) by substitution, deletion or addition of one or more amino acids and functionally identical or similar to the amino acid sequences set forth in (B4), and
(B6) amino acid sequences having at least 80% sequence identity to the amino acid sequences set forth in (B4).

6. The anti-CD47/anti-PD-L1 antibody according to any one of claims 1-5, wherein:
(1) the first heavy chain variable region comprises an amino acid sequence selected from the group consisting of:
(c1) an amino acid sequence set forth in SEQ ID NO: 30,
(c2) amino acid sequences derived from the amino acid sequences set forth in (c1) by substitution, deletion or addition of one or more amino acids and functionally identical or similar to the amino acid sequences set forth in (c1), and
(c3) amino acid sequences having at least 80% sequence identity to the amino acid sequences set forth in (c1); and
(2) the first light chain variable region comprises an amino acid sequence selected from the group consisting of:
(c4) an amino acid sequence set forth in SEQ ID NO: 33,
(c5) amino acid sequences derived from the amino acid sequences set forth in (c4) by substitution, deletion or addition of one or more amino acids and functionally identical or similar to the amino acid sequences set forth in (c4), and
(c6) amino acid sequences having at least 80% sequence identity to the amino acid sequences set forth in (c4).

7. The anti-CD47/anti-PD-L1 antibody according to any one of claims 1-6, comprising an anti-PD-L1 antibody or antigen-binding fragment thereof, wherein:
the anti-PD-L1 antibody or antigen-binding fragment thereof comprises a second heavy chain variable region and a second light chain variable region, wherein:
the second heavy chain variable region comprises an amino acid sequence selected from the group consisting of:
amino acid sequences set forth in SEQ ID NOs: 110, 111, 112, 113 and 114,
amino acid sequences derived from SEQ ID NO: 110, 111, 112, 113 or 114 by substitution, deletion or addition of one or more amino acids and functionally identical to SEQ ID NO: 110, 111, 112, 113 or 114, and
amino acid sequences comprising H2CDR1, H2CDR2 and H2CDR3 set forth in SEQ ID NOs: 75, 76 and 77 and having at least 85% sequence identity to SEQ ID NO: 110, 111, 112, 113 or 114; and
the second light chain variable region comprises an amino acid sequence selected from the group consisting of:
amino acid sequences set forth in SEQ ID NOs: 115, 116, 117 and 118,
amino acid sequences derived from SEQ ID NO: 115, 116, 117 or 118 by substitution, deletion or addition of one or more amino acids and functionally identical to SEQ ID NO: 115, 116, 117 or 118, and
amino acid sequences comprising L2CDR1, L2CDR2 and L2CDR3 set forth in SEQ ID NOs: 78, 79 and 80 and having at least 85% sequence identity to SEQ ID NO: 115, 116, 117 or 118; or
the second heavy chain variable region comprises an amino acid sequence selected from the group consisting of:
amino acid sequences set forth in SEQ ID NOs: 119, 120, 121, 122 and 123,
amino acid sequences derived from SEQ ID NO: 119, 120, 121, 122 or 123 by substitution, deletion or addition of one or more amino acids and functionally identical to SEQ ID NO: 119, 120, 121, 122 or 123, and
amino acid sequences comprising H2CDR1, H2CDR2 and H2CDR3 set forth in SEQ ID NOs: 87, 88 and 89 and having at least 85% sequence identity to SEQ ID NO: 119, 120, 121, 122 or 123; and
the second light chain variable region comprises an amino acid sequence selected from the group consisting of:
amino acid sequences set forth in SEQ ID NOs: 124, 125 and 126,
amino acid sequences derived from SEQ ID NO: 124, 125 or 126 by substitution, deletion or addition of one or more amino acids and functionally identical to SEQ ID NO: 124, 125 or 126, and
amino acid sequences comprising L2CDR1, L2CDR2 and L2CDR3 set forth in SEQ ID NOs: 90, 91 and 92 and having at least 85% sequence identity to SEQ ID NO: 124, 125 or 126.

8. The anti-CD47/anti-PD-L1 antibody according to any one of claims 1-6, comprising an anti-PD-L1 antibody or antigen-binding fragment thereof, wherein:
the anti-PD-L1 antibody or antigen-binding fragment thereof comprises a second heavy chain variable region and a second light chain variable region, wherein:
the second heavy chain variable region comprises an amino acid sequence selected from the group consisting of: an amino acid sequence set forth in SEQ ID NO: 112, amino acid sequences derived from SEQ ID NO: 112 by substitution, deletion or addition of one or more amino acids and functionally identical to SEQ ID NO: 112, and amino acid sequences comprising H2CDR1, H2CDR2 and H2CDR3 set forth in SEQ ID NOs: 75, 76 and 77 and having at least 85%, at least 90%, at least 95%, or at least 98% sequence identity to SEQ ID NO: 112; and the second light chain variable region comprises an amino acid sequence selected from the group consisting of: an amino acid sequence set forth in SEQ ID NO: 116, amino acid sequences derived from SEQ ID NO: 116 by substitution, deletion or addition of one or more amino acids and functionally identical to SEQ ID NO: 116, and amino acid sequences comprising L2CDR1, L2CDR2 and L2CDR3 set forth in SEQ ID NOs: 78, 79 and 80 and having at least 85%, at least 90%, at least 95%, or at least 98% sequence identity to SEQ ID NO: 116;
the second heavy chain variable region comprises an amino acid sequence selected from the group consisting of: an amino acid sequence set forth in SEQ ID NO: 123, amino acid sequences derived from SEQ ID NO: 123 by substitution, deletion or addition of one or more amino acids and functionally identical to SEQ ID NO: 123, and amino acid sequences comprising H2CDR1, H2CDR2 and H2CDR3 set forth in SEQ ID NOs: 87, 88 and 89 and having at least 85%, at least 90%, at least 95%, or at least 98% sequence identity to SEQ ID NO: 123; and the second light chain variable region comprises an amino acid sequence selected from the group consisting of: an amino acid sequence set forth in SEQ ID NO: 126, amino acid sequences derived from SEQ ID NO: 126 by substitution, deletion or addition of one or more amino acids and functionally identical to SEQ ID NO: 126, and amino acid sequences comprising L2CDR1, L2CDR2 and L2CDR3 set forth in SEQ ID NOs: 90, 91 and 92 and having at least 85%, at least 90%, at least 95%, or at least 98% sequence identity to SEQ ID NO: 126; or
the second heavy chain variable region comprises an amino acid sequence selected from the group consisting of: an amino acid sequence set forth in SEQ ID NO: 102, amino acid sequences derived from SEQ ID NO: 102 by substitution, deletion or addition of one or more amino acids and functionally identical to SEQ ID NO: 102, and amino acid sequences comprising H2CDR1, H2CDR2 and H2CDR3 set forth in SEQ ID NOs: 93, 94 and 95 and having at least 85%, at least 90%, at least 95%, or at least 98% sequence identity to SEQ ID NO: 102; and the second light chain variable region comprises an amino acid sequence selected from the group consisting of: an amino acid sequence set forth in SEQ ID NO: 106, amino acid sequences derived from SEQ ID NO: 106 by substitution, deletion or addition of one or more amino acids and functionally identical to SEQ ID NO: 106, and amino acid sequences comprising L2CDR1, L2CDR2 and L2CDR3 set forth in SEQ ID NOs: 96, 97 and 98 and having at least 85%, at least 90%, at least 95%, or at least 98% sequence identity to SEQ ID NO: 106.

9. The anti-CD47/anti-PD-L1 antibody according to any one of claims 1-8, wherein the antibody is a humanized antibody or a fully human antibody.

10. The anti-CD47/anti-PD-L1 antibody according to any one of claims 1-9, wherein the antibody is a bispecific antibody.

11. An isolated nucleic acid encoding the anti-CD47/anti-PD-L1 antibody according to any one of claims 1-10.

12. The nucleic acid according to claim 10, wherein
(1) the nucleotide sequence encoding the amino acid sequence of the first heavy chain variable region is set forth in SEQ ID NO: 36;
(2) the nucleotide sequence encoding the amino acid sequence of the first light chain variable region is set forth in SEQ ID NO: 39;
(3) the nucleotide sequence encoding the amino acid sequence of the second heavy chain variable region is set forth in SEQ ID NO: 127 or SEQ ID NO: 128; and
(4) the nucleotide sequence encoding the amino acid sequence of the second light chain variable region is set forth in SEQ ID NO: 129 or SEQ ID NO: 130.

13. An expression vector comprising the nucleic acid according to claim 11 or 12.

14. A host cell transformed with the expression vector according to claim 13, wherein the host cell is selected from the group consisting of prokaryotic cells and eukaryotic cells, preferably mammalian cells.

15. A method for producing the anti-CD47/anti-PD-L1 antibody according to any one of claims 1-10, comprising
expressing the antibody in the host cell according to claim 14, and
isolating the antibody from the host cell.

16. A pharmaceutical composition comprising the anti-CD47/anti-PD-L1 antibody according to any one of claims 1-10 and a pharmaceutically acceptable carrier.

17. Use of the anti-CD47/anti-PD-L1 antibody according to any one of claims 1-10 or the pharmaceutical composition according to claim 16 in the manufacture of a medicament for inhibiting CD47/PD-L1 activity, wherein the medicament is preferably used for treating hematological tumor, lymphoma, breast cancer, lung cancer, gastric cancer, intestinal cancer, esophageal cancer, ovarian cancer, cervical cancer, kidney cancer, bladder cancer, pancreatic cancer, glioma and/or melanoma.
